# EUROPEAN PATENT SPECIFICATION

(11) **EP 4 507 596 B1**
(45) Date of publication and mention of the grant of the patent: **02.09.2026**
(21) Application number: 23734798.4
(22) Date of filing: 26.05.2023
(51) Int. Cl.: A61B 17/32, A61B 17/3201, A61B 17/3209, A61B 18/14, A61B 17/3207, A61B 17/22, A61B 90/30

(54) **PERICARDIAL TRANSECTION DEVICES**
PERIKARDIALE TRANSEKTIONSVORRICHTUNGEN
DISPOSITIFS DE TRANSECTION PÉRICARDIQUE

(30) Priority: 31.05.2022 US 202263347528 P
(43) Date of publication of application: 19.02.2025
(73) Proprietor: Edwards Lifesciences Corporation, Irvine, CA 92614 (US)
(72) Inventor: KASHER, Yuval, 3079892 Caesarea (IL); SENESH, Gil, Laguna Beach, CA 92651 (US); MEYER-BRODNITZ, Gideon, 33073 Haifa (IL); BERWICK, Zachary, Christopher, Tustin, CA 92782 (US); ALBAGHDADI, Mazen, Saadi, Irvine, CA 92618 (US); LEV, Yaeer, E., Irvine, CA 92614 (US); KEEL, Allen, Jeong, Irvine, CA 92614 (US); AHMAD, Atiya, Makhdoom, Irvine, CA 92614 (US); BECERRA, Matthew, Michael, Irvine, CA 92614 (US); NICOLAU DA COSTA, Leonardo, Paim, Irvine, CA 92614 (US); STRAUSS, Yehoshua, 3079892 Caesarea (IL); HARPS, Daniel, M., Irvine, CA 92614 (US)
(74) Representative: Venner, Julia Ann
(86) International application number: PCT/US2023/023758
(87) International publication number: WO 2023/235263

(56) References cited:
- WO-A1-2021/046487
- US-A- 4 729 374
- US-A- 5 395 386
- US-A- 5 486 183
- US-A- 5 667 472
- US-A1- 2017 258 521
- US-A1- 2022 117 617
- US-B2- 10 307 179

## Description

### TECHNICAL FIELD

This disclosure is directed to devices and methods for cutting in the pericardium. The devices and methods are generally applicable to the treatment of heart failure, for example, heart failure with preserved ejection fraction (HFpEF) or reduced ejection fraction (HFrEF) by introducing one or more incision lengths in a pericardium, e.g., a parietal layer.

### BACKGROUND

Pericardial restraint is a normal physiologic process that becomes exaggerated, for example, in some patients with heart failure with preserved ejection fraction (HFpEF) and causes the right heart to run out of space when filling, thereby squeezing and over pressurizing the left heart during physical activity in these patients. The increased left heart pressure backs up into the lungs and causes these patients to experience significant breathing difficulties when trying to do minimal activity, (exertional dyspnea). Exertional dyspnea is the most common symptom in patients with HFpEF and the most common cause for admission to the hospital in patients with HF in general. Currently, there is no therapeutic option for patients with HFpEF that specifically targets pericardial restraint.

WO 2021/046487 describes systems and methods for treating cellulite including an apparatus that applies or a method involving separating septa to eliminate or reduce the appearance of cellulite. In one approach, an interventional tool is placed between tissue layers to engage and treat septa connecting tissue layers between which fat deposits are contained.

### SUMMARY

An aspect of the presently claimed invention is set out in the independent claim. Particular embodiments of this aspect are set out in the dependent claims. Any subject matter contained herein that does not fall within the scope of the appended claims is considered as being useful for understanding the invention. In particular, the methods of treatment disclosed herein do not form part of the presently claimed invention.

In an example, a medical device for creating elongated incisions in a pericardium is provided, the device comprising a flexible catheter comprising at least one lumen, a longitudinal axis, a proximal end, a distal end, and an incision device assembly having at least one opening is coupled to the distal end of the flexible catheter.

In another example, a method of improving a heart function in a heart of a subject having heart dysfunction is provided, the method comprising creating at least one incision length through a pericardium and reducing pressure exerted by the pericardium on a heart. In one example, the heart dysfunction is preserved ejection fraction.

In another example, a medical device is provided, the device comprising a catheter comprising a distal end, at least one lumen, and a longitudinal axis; an incision assembly coupled to the distal end of the catheter.

In an example embodiment, a medical device is provided. The medical device includes an incision assembly configured to be operably coupled to a distal end of a catheter. The incision assembly includes an incision opening defined along the incision assembly. The medical device also includes a retractable cutting apparatus disposed within the incision opening. The retractable cutting apparatus includes a first incision member including at least one cutting surface. The first incision member defines a first member distal end and a first member proximal end. The first member proximal end is rotatably fixed proximate to a distal end of the incision opening. The retractable cutting apparatus also includes a second incision member defining a second member distal end and a second member proximal end. The second member distal end is attached to the first incision member at a second member proximal end. The retractable cutting apparatus is configured to move between a retracted position and a deployed position. In an instance in which the retractable cutting apparatus is in the retracted position, the first incision member is within the incision opening. In an instance in which the retractable cutting apparatus is in the deployed position, as least a portion of the first incision member protrudes from the incision opening.

In some example embodiments, alone or in combination with any of the previous example embodiments, the at least one cutting surface of the first incision member includes at least one interior cutting surface or a cutting tip.

In some example embodiments, alone or in combination with any of the previous example embodiments, at least one of the at least one cutting surface of the first incision member is an electrode.

In some example embodiments, alone or in combination with any of the previous example embodiments, one or more of the at least one cutting surface of the first incision member comprises a monopolar electrode or a bipolar electrode.

In some example embodiments, alone or in combination with any of the previous example embodiments, the at least one cutting surface of the first incision member includes a blunt blade.

In some example embodiments, alone or in combination with any of the previous example embodiments, the second incision member includes at least one cutting surface and the at least one cutting surface faces the opposite direction of at least one of the at least one cutting surface of the first incision member.

In some example embodiments, alone or in combination with any of the previous example embodiments, the medical device includes one or more suction lumens defined along the incision assembly with the suction lumens providing a suction force in the direction in which the first incision member extends out of the incision opening.

In some example embodiments, alone or in combination with any of the previous example embodiments, the one or more suction lumens include a first suction lumen defined along a longitudinal axis of the incision assembly and a second suction lumen parallel to the first suction lumen in the longitudinal axis of the incision assembly.

In some example embodiments, alone or in combination with any of the previous example embodiments, the medical device also includes one or more orientation markers provided on the incision device with the one or more orientation markers indicating a cutting direction of the incision device, and the one or more orientation markers are radiopaque.

In some example embodiments, alone or in combination with any of the previous example embodiments, the medical device includes a camera provided via a camera lumen in the catheter with the camera being capable of providing one or more images of the retractable cutting apparatus.

In some example embodiments, alone or in combination with any of the previous example embodiments, the medical device includes one or more electrical wires configured to provide an electrical current to one or more of the at least one cutting surface of the first incision member with the one or more electrical providing a biasing force to the first incision member.

In some example embodiments, alone or in combination with any of the previous example embodiments, the electrical wire(s) include a torsion spring configured to provide the biasing force to the first incision member.

In some example embodiments, alone or in combination with any of the previous example embodiments, the medical device includes an actuator rod in communication with the second member distal end of the second incision member with the actuator rod being configured to move the first incision member and the second incision member.

In some example embodiments, alone or in combination with any of the previous example embodiments, the medical device includes a yoke attached to the second member distal end of the second incision member with the yoke being configured to receive the force from the actuator rod or a biasing force attached to the actuator rod.

In some example embodiments, alone or in combination with any of the previous example embodiments, the medical device includes a sheath configured to movably cover the incision opening.

In some example embodiments, alone or in combination with any of the previous example embodiments, the sheath is configured to provide a counterforce to the biasing force caused by the one or more electrical wires in an instance in which the sheath is at least partially covering the incision opening.

In some example embodiments, alone or in combination with any of the previous example embodiments, the at least one cutting surface of the first incision member includes a first interior cutting surface and a second interior cutting surface, with the first interior cutting surface and the second interior cutting surface both being electrodes, and the impedance between the first interior cutting surface and the second interior cutting surface indicating an instance in which a material is being cut by the first interior cutting surface and the second interior cutting surface.

In some example embodiments, alone or in combination with any of the previous example embodiments, the medical device includes a controller engaged to the incision assembly or retractable cutting apparatus.

In some example embodiments, alone or in combination with any of the previous example embodiments, the controller is configured to provide at least one of suction to the one or more suction lumens, movement of the first incision member and the second incision member between the retracted position and the deployed position, movement of the incision assembly, energy to the incision device, or movement of one or more stabilizing members.

In another example embodiment, a method of manipulating a medical device is provided. The method includes providing a medical device of any one of the previous claims engaged with a controller. The method also includes controlling at least one of supplying suction to the one or more suction lumens, movement of the first incision member and the second incision member between the retracted position and the deployed position, movement of the incision assembly, supplying energy to the incision device, or movement of one or more stabilizing members.

In another example embodiment, a medical device for creating elongated incisions within a pericardium is provided. The medical device includes a catheter including at least one lumen, a longitudinal axis, a proximal end, and a distal end. The medical device also includes an incision device operably coupled to the distal end of the catheter. The medical device further includes a biasing member operably coupled to the incision device configured to laterally project at least a portion of the incision device through the at least one opening of the distal portion of the catheter.

In some example embodiments, alone or in combination with any of the previous example embodiments, the catheter is steerable.

In some example embodiments, alone or in combination with any of the previous example embodiments, at least a portion of the catheter is radiopaque.

In some example embodiments, alone or in combination with any of the previous example embodiments, the at least one lumen includes a guidewire lumen extending through the incision device.

In some example embodiments, alone or in combination with any of the previous example embodiments, at least a portion of the incision device is radiopaque.

In some example embodiments, alone or in combination with any of the previous example embodiments, the incision device includes a rigid housing and a cutting surface received by the rigid housing. In some example embodiments, alone or in combination with any of the previous example embodiments, the rigid housing is metal, polymer, ceramic, or combinations.

In some example embodiments, alone or in combination with any of the previous example embodiments, the biasing member is positioned in the incision device. In some example embodiments, alone or in combination with any of the previous example embodiments, the biasing member exerts a rotary force or torque to the cutting surface.

In some example embodiments, alone or in combination with any of the previous example embodiments, the medical device also includes an actuator coupled to the biasing member.

In some example embodiments, alone or in combination with any of the previous example embodiments, the medical device also includes a guidewire slidably positioned within the guidewire lumen.

In some example embodiments, alone or in combination with any of the previous example embodiments, the medical device also includes an atraumatic tapered tip comprising a lumen. In some example embodiments, alone or in combination with any of the previous example embodiments, the atraumatic tapered tip lumen slidably receives the guidewire. In some example embodiments, alone or in combination with any of the previous example embodiments, at least a portion of the atraumatic tapered tip is radiopaque.

In some example embodiments, alone or in combination with any of the previous example embodiments, the medical device also includes a sheath with a distal end being slidably locatable on the catheter. In some example embodiments, alone or in combination with any of the previous example embodiments, at least a portion of the distal end of the sheath is radiopaque. In some example embodiments, alone or in combination with any of the previous example embodiments, the sheath further includes at least one opening adjacent the distal end of the sheath.

In some example embodiments, alone or in combination with any of the previous example embodiments, the distal end of the sheath is traversable along the catheter to align with the at least one opening of the catheter. In some example embodiments, alone or in combination with any of the previous example embodiments, the at least one opening of the sheath is traversable along the catheter to cover the at least one opening of the catheter or to uncover the at least one opening of the catheter.

In some example embodiments, alone or in combination with any of the previous example embodiments, at least a portion of the distal end of the sheath and at least a portion of the at least one opening of the catheter are radiopaque to align the distal end of the sheath and the at least one opening of the catheter. In some example embodiments, alone or in combination with any of the previous example embodiments, at least a portion of a circumference of the at least one opening of the sheath and at least a portion of a circumference of the at least one opening of the catheter are radiopaque to align their respective openings.

In some example embodiments, alone or in combination with any of the previous example embodiments, the at least one opening of the sheath is traversable to align with the at least one opening of the catheter, allowing the cutting surface to laterally project the through both the sheath and the catheter. In some example embodiments, alone or in combination with any of the previous example embodiments, the at least one opening of the sheath is traversable to align with the at least one opening of the catheter, allowing the actuator to cause the cutting surface to laterally project the through both the sheath and the catheter.

In some example embodiments, alone or in combination with any of the previous example embodiments, the medical device also includes one or more stabilizing members located adjacent the at least one opening of the catheter. In some example embodiments, alone or in combination with any of the previous example embodiments, the one or more stabilizing members reversibly projects laterally about 120 degrees radially apart about the catheter.

In some example embodiments, alone or in combination with any of the previous example embodiments, the one or more stabilizing member is a wire, loop, or shape-memory metal. In some example embodiments, alone or in combination with any of the previous example embodiments, the one or more stabilizing member is one or more inflatable structures.

In some example embodiments, alone or in combination with any of the previous example embodiments, the distal end of the sheath is traversable along the catheter to uncover the at least one opening of the catheter allowing the one or more stabilizing members to laterally project through the one or more openings of the catheter.

In some example embodiments, alone or in combination with any of the previous example embodiments, the distal end of the sheath concurrently or sequentially allows the cutting surface and the one or more stabilizing members to laterally project through the one or more openings of the catheter. In some example embodiments, alone or in combination with any of the previous example embodiments, the one or more stabilizing members are operably coupled to the actuator.

In some example embodiments, alone or in combination with any of the previous example embodiments, the one or more openings of the sheath are traversable along the catheter allowing the actuator to cause the one or more stabilizing members to laterally project through both the catheter and the sheath. In some example embodiments, alone or in combination with any of the previous example embodiments, the actuator concurrently or sequentially laterally projects the incision device and the one or more stabilizing members through the one or more openings of the catheter and the one or more openings of the sheath.

In some example embodiments, alone or in combination with any of the previous example embodiments, at least a portion of the incision device comprises at least one electrode. In some example embodiments, alone or in combination with any of the previous example embodiments, at least a portion of the cutting surface includes an electrode. In some example embodiments, alone or in combination with any of the previous example embodiments, the electrode is a wire.

In some example embodiments, alone or in combination with any of the previous example embodiments, the wire is shaped as one or more arcs projecting laterally through the catheter along the longitudinal axis.

In some example embodiments, alone or in combination with any of the previous example embodiments, the cutting surface includes a scalpel. In some example embodiments, alone or in combination with any of the previous example embodiments, at least a portion of the scalpel includes an electrode.

In some example embodiments, alone or in combination with any of the previous example embodiments, the at least one electrode is electrically couplable to a source of radiofrequency energy or electrical current sufficient to cut, separate, scissor, or evaporate a portion of the parietal layer.

In some example embodiments, alone or in combination with any of the previous example embodiments, the medical device also includes a second electrode adjacent to the incision device. In some example embodiments, alone or in combination with any of the previous example embodiments, the second electrode is operably coupled to a source of radiofrequency energy or electrical current.

In some example embodiments, alone or in combination with any of the previous example embodiments, the cutting surface when laterally projected, faces away from the distal end and towards the proximal end of the catheter (i.e., proximally-facing). In some example embodiments, alone or in combination with any of the previous example embodiments, at least a portion of cutting surface is reversibly adjustable laterally relative to the longitudinal axis of the catheter between a range of angles.

In some example embodiments, alone or in combination with any of the previous example embodiments, the range of angles are reversibly adjustable for providing a scissor cut action.

In some example embodiments, alone or in combination with any of the previous example embodiments, the medical device also includes at least one nerve detection device. In some example embodiments, alone or in combination with any of the previous example embodiments, the at least one nerve detection device is located on the catheter. In some example embodiments, alone or in combination with any of the previous example embodiments, the at least one nerve detection device is located adjacent the incision device. In some example embodiments, alone or in combination with any of the previous example embodiments, the at least one nerve detection device is located on the dilator. In some example embodiments, alone or in combination with any of the previous example embodiments, the at least one nerve detection device is located on the cutting surface.

In some example embodiments, alone or in combination with any of the previous example embodiments, the medical device also includes at least one nerve stimulation device. In some example embodiments, alone or in combination with any of the previous example embodiments, the at least one nerve stimulation device is located on the catheter. In some example embodiments, alone or in combination with any of the previous example embodiments, the at least one nerve stimulation device is located adjacent the incision device. In some example embodiments, alone or in combination with any of the previous example embodiments, the at least one nerve stimulation device is located on the dilator. In some example embodiments, alone or in combination with any of the previous example embodiments, the at least one nerve stimulation device is located on the cutting surface.

In some example embodiments, alone or in combination with any of the previous example embodiments, a kit is provided with the medical device of any one of the example embodiments, a sheath, a guidewire, and a dilator.

In another example, a medical device is provided. The medical device includes a flexible catheter including a distal end, at least one lumen, and a longitudinal axis. The medical device also includes an incision assembly coupled to the distal end of the catheter. The medical device further includes an atraumatic tapered tip.

In some example embodiments, alone or in combination with any of the previous example embodiments, the flexible catheter is steerable. In some example embodiments, alone or in combination with any of the previous example embodiments, at least a portion of the flexible catheter is radiopaque. In some example embodiments, alone or in combination with any of the previous example embodiments, the at least one lumen includes a guidewire lumen.

In some example embodiments, alone or in combination with any of the previous example embodiments, the incision assembly includes a housing, an opening in the housing, and a cutting surface. In some example embodiments, alone or in combination with any of the previous example embodiments, at least a portion of the incision assembly is radiopaque. In some example embodiments, alone or in combination with any of the previous example embodiments, at least a portion of a housing of the incision assembly is radiopaque.

In some example embodiments, alone or in combination with any of the previous example embodiments, the incision assembly includes a lumen operably coupled to the guidewire lumen. In some example embodiments, alone or in combination with any of the previous example embodiments, the atraumatic tapered tip includes a lumen operably coupled to the guidewire lumen.

In some example embodiments, alone or in combination with any of the previous example embodiments, the flexible catheter, incision assembly, and atraumatic tapered tip slidably receive a guidewire. In some example embodiments, alone or in combination with any of the previous example embodiments, the incision assembly is configured for introduction over the guidewire.

In some example embodiments, alone or in combination with any of the previous example embodiments, the incision assembly has a first configuration with the cutting surface received in the incision assembly, and a second configuration wherein the cutting surface is projected laterally outward from the incision assembly.

In some example embodiments, alone or in combination with any of the previous example embodiments, the incision assembly is operably coupled to a controller, the controller positioned adjacent a proximal end of the flexible catheter.

In some example embodiments, alone or in combination with any of the previous example embodiments, the incision assembly includes a biasing member operably coupled to the cutting surface. In some example embodiments, alone or in combination with any of the previous example embodiments, the biasing member is operably coupled to the controller.

In some example embodiments, alone or in combination with any of the previous example embodiments, the biasing member exerts a force or torque to the cutting surface. In some example embodiments, alone or in combination with any of the previous example embodiments, the force or torque is rotary. In some example embodiments, alone or in combination with any of the previous example embodiments, the biasing member is a spring or band. In some example embodiments, alone or in combination with any of the previous example embodiments, the spring is a torsion spring or compression spring. In some example embodiments, alone or in combination with any of the previous example embodiments, the spring is a torsion spring in combination with a compression spring. In some example embodiments, alone or in combination with any of the previous example embodiments, the torsion spring is a double torsion spring.

In some example embodiments, alone or in combination with any of the previous example embodiments, the incision assembly further includes a pivot pin, the pivot pin securing the torsion spring, alone or in combination with the compression spring, and a proximal end of the scalpel within the incision assembly.

In some example embodiments, alone or in combination with any of the previous example embodiments, the double torsion spring straddles the cutting surface. In some example embodiments, alone or in combination with any of the previous example embodiments, the cutting surface is biased to rotate laterally outward through the at least one opening of the flexible catheter. In some example embodiments, alone or in combination with any of the previous example embodiments, the cutting surface is biased to pivotably rotate laterally outward from the flexible catheter.

In some example embodiments, alone or in combination with any of the previous example embodiments, the cutting surface has a rounded distal end. In some example embodiments, alone or in combination with any of the previous example embodiments, the cutting surface has a pointed distal end, the pointed distal end configured to puncture the pericardial tissue from within a pericardial space.

In some example embodiments, alone or in combination with any of the previous example embodiments, the cutting surface is angled between its distal end and its proximal end. In some example embodiments, alone or in combination with any of the previous example embodiments, the angle between the distal end and the proximal end of the cutting surface is acute. In some example embodiments, alone or in combination with any of the previous example embodiments, the cutting surface forms an acute angle between its distal end and an outer surface of the flexible catheter.

In some example embodiments, alone or in combination with any of the previous example embodiments, the angle between the cutting surface and the outer surface of the flexible catheter is configured to receive at least a portion of pericardial tissue.

In some example embodiments, alone or in combination with any of the previous example embodiments, the housing includes an anti-buckle mechanism. In some example embodiments, alone or in combination with any of the previous example embodiments, the anti-buckle mechanism includes a spring axially aligned with the longitudinal axis of the flexible catheter.

In some example embodiments, alone or in combination with any of the previous example embodiments, the incision assembly further includes a conductive wire operably coupled to the housing. In some example embodiments, alone or in combination with any of the previous example embodiments, the conductive wire is operably coupled to the controller.

In some example embodiments, alone or in combination with any of the previous example embodiments, at least a portion of either side of the cutting surface is, independently, coupled to a source of current or RF.

In some example embodiments, alone or in combination with any of the previous example embodiments, the incision assembly further includes at least one stabilizirefng member. In some example embodiments, alone or in combination with any of the previous example embodiments, the at least one stabilizing member is operably coupled to the controller. In some example embodiments, alone or in combination with any of the previous example embodiments, the at least one stabilizing members reversibly projects laterally from the incision assembly. In some example embodiments, alone or in combination with any of the previous example embodiments, the controller concurrently or sequentially laterally projects the cutting surface and/or the at least one stabilizing member through the one or more openings of the flexible catheter.

In some example embodiments, alone or in combination with any of the previous example embodiments, the at least one stabilizing members reversibly projects laterally about 120 degrees radially apart about the incision assembly. In some example embodiments, alone or in combination with any of the previous example embodiments, the at least one stabilizing member is a wire, loop, shape-memory metal, or a drawn filled tube (DFT) wire. In some example embodiments, alone or in combination with any of the previous example embodiments, the at least one stabilizing member is an inflatable structure.

In some example embodiments, alone or in combination with any of the previous example embodiments, the medical device also includes at least one nerve detection device. In some example embodiments, alone or in combination with any of the previous example embodiments, the at least one nerve detection device is located on the flexible catheter. In some example embodiments, alone or in combination with any of the previous example embodiments, the at least one nerve detection device is located adjacent the incision assembly. In some example embodiments, alone or in combination with any of the previous example embodiments, the at least one nerve detection device is located on the introducer. In some example embodiments, alone or in combination with any of the previous example embodiments, the at least one nerve detection device is located on the cutting surface.

In some example embodiments, alone or in combination with any of the previous example embodiments, the medical device also includes at least one nerve stimulation device. In some example embodiments, alone or in combination with any of the previous example embodiments, the at least one nerve stimulation device is located on the flexible catheter. In some example embodiments, alone or in combination with any of the previous example embodiments, the at least one nerve stimulation device is located adjacent the incision assembly. In some example embodiments, alone or in combination with any of the previous example embodiments, the at least one nerve stimulation device is located on the introducer. In some example embodiments, alone or in combination with any of the previous example embodiments, the at least one nerve stimulation device is located on the cutting surface.

In some example embodiments, alone or in combination with any of the previous example embodiments, at least a portion of the atraumatic tapered tip is radiopaque.

In some example embodiments, alone or in combination with any of the previous example embodiments, the cutting surface faces away from the distal end and towards the proximal end of the flexible catheter. In some example embodiments, alone or in combination with any of the previous example embodiments, the incision assembly is configured to reversibly pivot the cutting surface from a projected configuration to a retracted configuration for providing a scissor-like action.

In some example embodiments, alone or in combination with any of the previous example embodiments, the incision assembly has a first configuration with the cutting surface covered by the distal end of the sheath, and a second configuration wherein the cutting surface is laterally projected when the sheath is longitudinally traversed from the incision assembly.

In some example embodiments, alone or in combination with any of the previous example embodiments, at least a portion of the distal end of the sheath is radiopaque to align the distal end of the sheath and the incision assembly.

In some example embodiments, alone or in combination with any of the previous example embodiments, at least a portion of the cutting surface includes an electrode, and the cutting surface is pivotably projectable so that the electrode is exposed to pericardial tissue. In some example embodiments, alone or in combination with any of the previous example embodiments, the cutting surface includes an electrode, and the cutting surface is partially pivotably projectable so that at least a portion of the electrode is concealed from pericardial tissue.

In some example embodiments, alone or in combination with any of the previous example embodiments, the electrode is a wire. In some example embodiments, alone or in combination with any of the previous example embodiments, the wire is shaped as one or more arcs projecting laterally from the flexible catheter along the longitudinal axis.

In another example, a medical device is provided. The medical device includes an elongated body comprising a distal end, at least one lumen, and a longitudinal axis. The medical device also includes an incision assembly coupled to the distal end of the catheter. The medical device further includes a first radiopaque marker positioned about the elongated body. The medical device still further includes a second radiopaque marker positioned about the incision assembly. The medical device also includes a first configuration where the first radiopaque marker and the second radiopaque marker are in proximity to each other and a second configuration where the first radiopaque marker and the second radiopaque marker are in spatially separated from each other.

In some example embodiments, alone or in combination with any of the previous example embodiments, the incision assembly is pivotably connected to the elongated body.

In some example embodiments, alone or in combination with any of the previous example embodiments, the incision assembly includes an incision member. In some example embodiments, alone or in combination with any of the previous example embodiments, the incision member is a blade, electrode, or a combination of blade and electrode.

In some example embodiments, alone or in combination with any of the previous example embodiments, the incision assembly includes an atraumatic tapered tip. In some example embodiments, alone or in combination with any of the previous example embodiments, the atraumatic tapered tip is an electrode.

In some example embodiments, alone or in combination with any of the previous example embodiments, the incision assembly is operably coupled to a controller. In some example embodiments, alone or in combination with any of the previous example embodiments, the device is sterilized.

### BRIEF DESCRIPTION OF THE DRAWINGS

In order to understand and to see how the present disclosure may be carried out in practice, examples will now be described, by way of non-limiting examples only, with reference to the accompanying drawings, in which:
FIG. 1A is a sectional view of a 4-chambered heart.
FIG. 1B is an enlarged view of section 1B of FIG. 1A depicting the layers of the heart wall, including the pericardial cavity.
FIG. 1C is a further enlarged view of section 1C of FIG. 1A depicting the serosal, visceral, fibrous layers and adipose tissue of the parietal pericardium, including the pericardial cavity.
FIG. 2A is a depiction of an exemplary device in a deployed configuration, as disclosed and described herein.
FIG. 2B is a partial cut-away view of section 2B of FIG. 2A, digitally depicting pericardial tissue transection device, as disclosed and described herein.
FIGs. 3A and 3B are a partial cut-away view of a pericardial tissue transection device, as disclosed and described herein.
FIGs. 4A, 4B and 4C depict an exemplary pericardial tissue transection device shown in a first, second, and third configuration, as disclosed and described herein.
FIGs. 5A and 5B depict an exemplary pericardial tissue transection device in a first and second configuration, as disclosed and described herein.
FIG. 5C depicts a digital rendering of the exemplary pericardial tissue transection device of FIG. 5B, shown in a deployed configuration.
FIG. 6A depicts an exemplary pericardial tissue transection device shown in a deployed active scissoring configuration.
FIG. 6B, depict digital renderings of the exemplary pericardial tissue transection device of FIG. 6A in a first and second state of the active configuration, as disclosed and described herein.
FIGs. 7A, 7B and 7C depict first side, second side, and partial cut-away views, respectively, of an exemplary pericardial tissue transection device, as disclosed and described herein.
FIGs. 7D and 7E depict partial cut-away and exploded views, respectively, of the exemplary pericardial tissue transection device of FIG. 7A, as disclosed and described herein.
FIGs. 7F, 7G, and 7H depict a method of creating an incision in the pericardial layer using the exemplary pericardial tissue transection device of FIG. 7A, as disclosed and described herein.
FIGs. 8A, 8B and 8C depict an exemplary pericardial tissue transection device shown in a first and second configuration, where FIG. 8C is a view of FIG. 8B rotated 90 degrees, as disclosed and described herein.
FIGs. 9A, 9B and 9C depict an exemplary pericardial tissue transection device shown in a first and second configuration, where FIG. 9C is a view of FIG. 9B rotated 90 degrees, as disclosed and described herein.
FIGs. 10A, 10B and 10C depict a method of creating an incision in the pericardial layer using an exemplary device as disclosed and described herein.
FIGs. 11A, 11B and 11C depict a method of creating an incision in the pericardial layer using an exemplary device as disclosed and described herein.
FIGs. 12A, 12B and 12C depict an exemplary pericardial tissue transection device shown in a first, second and third configuration creating an incision in the pericardial layer, as disclosed and described herein.
FIG. 13A depicts an exemplary pericardial tissue transection device as disclosed and described herein.
FIG. 13B depicts another exemplary pericardial tissue transection device as disclosed and described herein.
FIGs. 13C, 13D, and 13E depict the exemplary pericardial tissue transection device of FIG. 13A, shown in a first, second and third configuration, as disclosed and described herein.
FIGs. 14A, 14B and 14C depict a method of creating an incision in the pericardial layer using the pericardial tissue transection device of FIG. 13A, as disclosed and described herein.
FIGs. 14D, 14E and 14F depict a method of creating an elongated incision in the pericardial layer using the pericardial tissue transection device of FIG. 13B, as disclosed and described herein
FIGs. 15A and 15B depict an exemplary introducer/controller devices for delivering and operating the pericardial tissue transection devices disclosed and described herein.
FIG. 15C depicts another exemplary introducer/controller device for delivering and operating the pericardial tissue transection devices disclosed and described herein.
FIG. 16 depicts a visualization system for use in a catheter device in combination with the presently disclosed pericardial layer tissue transection devices.
FIG. 17 is a simplified diagram of a catheter approach to the pericardial cavity, as disclosed and described herein.
FIG. 18 is a simplified diagram of an alternative catheter approach to the pericardial cavity, as disclosed and described herein.
FIG. 19 is a simplified diagram of a parietal layer incision length and cut path as disclosed and described herein.
FIGs. 20A-20H depict various views of an example pericardial tissue transection device, as disclosed and described herein.
FIG. 21 depicts the various lumens in transection device to be coupled to a catheter in accordance with various embodiments, as disclosed and described herein.
FIG. 22 illustrates an example controller with an incision assembly disposed thereon in accordance with various embodiments, as disclosed and described herein.
FIG. 23 illustrates an exploded view of an incision assembly in accordance with various embodiments, as disclosed and described herein.
FIGs. 24A, 24B, and 24C illustrate various views of a first incision member in accordance with various embodiments, as disclosed and described herein.
FIG. 25A illustrates an exploded view of another example incision assembly in accordance with various embodiments, as disclosed and described herein.
FIG. 25B illustrates a zoomed-in view of the connection of the first incision member in accordance with various embodiments, as disclosed and described herein.
FIG. 25C illustrates the incision assembly of FIG. 25A in the deployed position in accordance with various embodiments, as disclosed and described herein.
FIG. 26A is an exploded view of another example incision assembly in accordance with various embodiments, as disclosed and described herein.
FIG. 26B illustrates another view of the incision assembly of FIG. 26A in accordance with various embodiments, as disclosed and described herein.
FIG. 27 illustrates the connection between electrical wires and various cutting surfaces in accordance with various embodiments, as disclosed and described herein.
FIGs. 28A, 28B, and 28C illustrate various exploded views of the first incision member in accordance with various embodiments, as disclosed and described herein.

### DETAILED DESCRIPTION

The present disclosure provides for a catheter-based therapy referred to as catheter alleviation of pericardial restraint (TAPR) that can reduce pericardial restraint by incising or opening the pericardium with the intention of improving patient outcomes with heart dysfunction, for example, HFpEF or HFrEF and reducing HF readmissions related thereto. The present disclosure, in one example, provides a pericardial transection device with a concealed/proximally-facing cutting surface 105 for accessing and modifying a subject's pericardium for relieving pericardial restraint and/or resolving a heart dysfunction. The present disclosure further provides for methods of treating heart dysfunction using the presently disclosed device.

As used herein the phrase "pericardial space" and pericardial cavity are used interchangeably and are inclusive of their ordinary and customary meaning to one of ordinary skill in medical and surgical arts, for example, a space, cavity, or liquid medium generally disposed between the parietal pericardium and visceral pericardium of a mammalian heart.

As used herein the phrase "pericardial tissue" is inclusive of its ordinary and customary meaning to one of ordinary skill in medical and surgical arts, for example, tissue associated with the pericardium.

As used herein, unless otherwise specified, the phrase "parietal layer" comprises at least the serosal layer of the parietal pericardium and the fibrous layer of the parietal pericardium, and optionally adipose tissue contained between, below, above, or within said layers. Further, the phrase "parietal layer" is inclusive of the ordinary and customary meaning to one of ordinary skill in medical and surgical arts, for example tissue layers generally disposed the adjacent to and including adipose tissue within and outside the pericardial cavity and superficial to the visceral layer of the pericardium.

As used herein the phrase "cutting surface" is inclusive of one or more of an edge of a sharpened blade or the surface of an electrode configured to receive sufficient current or radio frequency energy (RF) to ablate, burn, vaporize, or separate tissue. A cutting surface can be inclusive of both a sharpened edge and an electrode.

As used herein the phrase "reverse cutting" and "pull-back cutting" are used interchangeably and refer to methods involving the presentation of a cutting surface to tissue, the cutting surface adjacent a distal end of a catheter device or catheter, and the application of a directional force sufficient to cut or separate the tissue, the force being substantially in a direction towards the proximal end of the catheter device or catheter, for example, by pulling the catheter device or catheter while the cutting surface is engaged with the tissue.

It should be understood that the term "cutting" used herein refers to tissue disruption, for example, a sharp-cutting incision of the type associated with a knife blade such as a scalpel blade, or an electrosurgical device that provides electrical current to an electrically conductive material or electrode sufficient to disrupt tissue. The term "cutting" used herein includes "filet", "slicing", and the like.

As used herein the phrase "incision length" is inclusive of a non-zero distance of a cut or incision, for example, beginning at a first point, e.g., a target point, and terminating at a second point, e.g., an access point. An incision length can be linear, non-linear, or a plurality of linear and/or non-linear lengths that intersect or do not intersect about a curved or non-planar surface, such a heart.

As used herein the phrase "reducing pressure" and "reducing restraint" are inclusive of their ordinary and customary meaning of one to ordinary skill in medical and surgical arts.

As used herein the phrase "preserved ejection fraction" is inclusive of the ordinary and customary meaning to one of ordinary skill in medical and surgical arts, for example, a clinical syndrome in which patients display signs and symptoms of heart failure as the result of high left ventricular (LV) filling pressure despite normal or near normal left ventricle (LV) ejection fraction (LVEF; ≥50 percent).

As used herein the phrase "reduced ejection fraction" is inclusive of the ordinary and customary meaning to one of ordinary skill in medical and surgical arts, for example, impairment of ventricular filling or ejection of blood or both, with a clinical syndrome in which patients display left ventricular ejection fraction (LVEF) of 40% or less and are accompanied by progressive left ventricular dilatation and adverse cardiac remodeling and/or mitral valve dysfunction.

As used herein the phrase "heart dysfunction" is inclusive of the ordinary and customary meaning to one of ordinary skill in medical and surgical arts, for example, heart failure, congestive heart failure, HFpEF, or HFrEF.

As used herein the phrase "incision device" is inclusive of a device with a cutting surface, for example an edge of a blade or a surface of an energized electrode.

As used herein the phrase "pericardial incision assembly" and "incision assembly" are used interchangeably and refer to an assemblage that includes an incision device.

As used herein the phrase "transcatheter device" is inclusive of a catheter configured with at least one lumen comprising a medical instrument, device, or component thereof, for example, an incision device, guidewire, optical fiber, contrast fluid lumen, stabilizing members, etc.

As used herein, the terms "first," "second," and the like are only used to describe elements as they relate to one another, and are in no way meant to recite specific orientations of an article or apparatus, to indicate or imply necessary or required orientations of an article or apparatus, to indicate or imply necessary or required configurations of an article or apparatus, or to specify how an article or apparatus described herein will be used, deployed, transitioned from different configurations, or positioned in use.

As used herein, when an element is referred to as being "adjacent" and "coupled" when referring to two structures or layers, the two structures or layers are in proximity with one another with no intervening open space between them.

As used herein, when an element is referred to as being "coupled" or "adjacent" to another element, the two elements or structures are in proximity with one another, however, other elements or intervening elements may be present.

As used herein, when an element is referred to as being "directly coupled" or "directly adjacent" to another element, other elements or intervening elements are not present.

As used herein, term "operably coupled", includes direct coupling and indirect coupling via another component, element, circuit, or structure and/or indirect coupling between items via an intervening item.

As used herein the phrase "nerve stimulation device" is inclusive of a device capable of applying an electrical potential to a nerve and to cause an observable effect that is directly or indirectly correlated to the applied potential, for example a pacing probe stimulating a phrenic nerve and causing an observable breathing disruption.

As used herein the phrase "nerve detecting device" is inclusive of a device capable of establishing a location or locale of at least part of a nerve and providing location or proximity information with no or substantially no physical effect or stimulus on the nerve, for example, an impedance sensor for detecting an electrical field generated by a nerve and to correlate, directly or indirectly, the location or proximity of the nerve relative to the impedance sensor.

As used herein the term "actuator" is inclusive of a mechanism for triggering an action.

As used herein the term "controller" is inclusive of a device having an actuator.

As used herein the phrase "biasing member" is inclusive of a device configurable in a stored energy state and a released energy state, for example, a spring.

As used herein the phrase "stabilizing member" is inclusive of a device configurable to impart stability and/or securement of a device to or within a structure, such as for example, stabilizing or securing a cutting surface positioned in a pericardial cavity from rolling, twisting, buckling and/or oscillating prior to or during use.

As used herein the phrase "puncturing tip" is inclusive of an atraumatic object suitable for puncturing or penetrating tissue without substantial trauma to or bleeding from the vicinity of the picture or penetration.

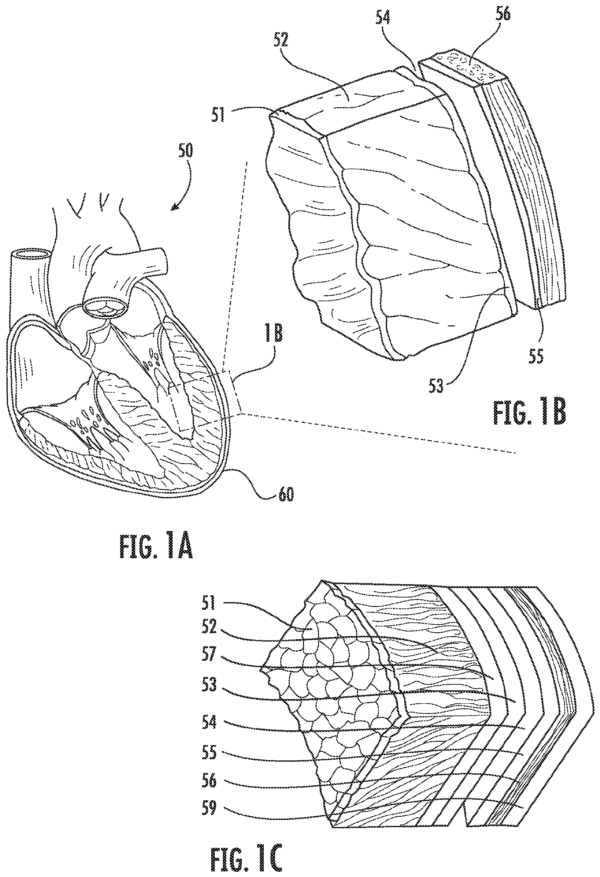

With reference to FIGs. 1A, 1B, 1C, and sections 1B, 1C, layers of a heart wall of a heart 50, from inside-out, being the endocardium 51, the myocardium 52, epicardial adipose tissue 57, the visceral layer 53 of the serous pericardium, the pericardial cavity 54, the parietal layer 55 of the serous pericardium, and the fibrous pericardium 56, and pericardial adipose tissue 59, are depicted. In one example, the presently disclosed devices are configured for introduction to the pericardial cavity 54 and for cutting tissue layers generally disposed adjacent to and including adipose tissue within and outside the pericardial cavity and superficial to the visceral layer 53 of the pericardium.

The presently disclosed pericardial tissue transection devices includes a perforating or puncturing portion designed to initially puncture the pericardial membrane. A guidewire, knife, or electrical current may be used to form the perforation or puncturing of the pericardial membrane to allow access of the pericardial transection device to the pericardial cavity. Once the pericardial membrane is punctured, an incision assembly adjacent a distal end of a catheter or catheter is manipulated to a location within the pericardial cavity, and an incision member is allowed to engage with the pericardial tissue and an incision length is created. The incision member may alternatively, or in combination with a sharp edge, utilize RF energy to facilitate ease of incising and for providing some hemostasis of the pericardial membrane. The incision length can be made, either subphxoidally, or transvascularly, by advancing an incision device, from the initial pericardial cavity entry location with cutting to a distal location (forward cutting). Alternatively, the incision length can be made by traversing the incision device from the initial pericardial cavity entry location to a distal location and then reversing back towards the entry location with cutting (reverse cutting). Thus, the presently disclosed pericardial transection devices are configured for either forward or reverse cutting modalities.

Several pericardial tissue transection device examples are shown in the attached figures and disclosed herein. Hereinafter, the phrase "pericardial tissue transection device" and "transection device" shall be used interchangeably. Each transection device would be first introduced into the pericardial space via a transvascular approach or subxiphoid approach.

With general reference to the Figures, in one example the presently disclosed medical device comprises a flexible catheter 129 comprising a distal end, at least one lumen, and a longitudinal axis; an incision assembly 101 coupled to the distal end of the catheter; and an atraumatic tip (e.g., introducer 115) coupled to and projecting from the incision assembly 101. In one example, example the presently disclosed medical device is deployed with the use of a hollow needle/introducer/dilator kit. In one example, at least a portion of the flexible catheter 129 tip is radiopaque. In one example, at least a portion of the incision assembly 101 tip is radiopaque.

In an alternative example, the medical device for creating elongated incisions in a pericardium is described. The device comprising a flexible catheter 129 comprising at least one lumen, a longitudinal axis, a proximal end, and a distal end along the longitudinal axis. In one example, an incision device assembly 101 having at least one opening 136, is coupled to the flexible catheter 129. A biasing member 117 is operably coupled to the incision device to laterally project at least a portion of the incision device through the at least one opening of the incision device assembly 101. In one example, a dilator adjacent the distal end of the flexible catheter 129 is employed. In one example, the device provides for creating elongated incisions of an incision length in a parietal layer of the pericardium. The incision device comprises at least one cutting surface. The cutting surface can be a knife or scalpel or an electrode. In one example, at least a portion of the opening 136 is radiopaque.

The cutting surface 105 may comprise a blade 103 and/or an electrode (e.g., cutting tip 125). In one example, the presently disclosed device has a user-controlled concealed/proximally-facing cutting surface 105, the cutting surface 105 controlled by a controller and/or actuator

The cutting surface 105 may be (or include) a blade 103, one or more RF electrodes for electrosurgical cutting, or both. In one example, a single RF electrode (e.g., cutting tip 125) may be provided at/near the hinge of the cutting surface 105 - e.g., a monopolar arrangement.

In one example, one electrode may be at/on the cutting surface 105, and another may be at/on the body of the device - e.g., a bipolar arrangement. In one example, the blade 103 itself may serve as an RF electrode. In one example, RF is used to sufficiently weaken the tissue, and the cutting surface 105 is not a blade 103 but nonetheless can clamp or burn the RF-weakened tissue sufficiently to cut it.

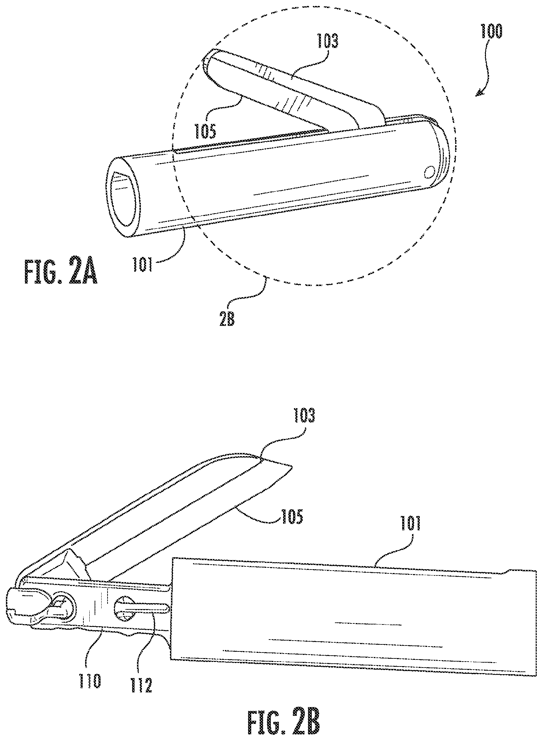

With reference to FIGs. 2A-2B, the cutting surface 105 of device 100 may be biased to be in its extended configuration as shown, with the device having a cable 112 thru housing 110 that is tensioned to retract blade 103 into the incision assembly 101 as to maintain the cutting surface 105 in the housing of the device in a retracted configuration.

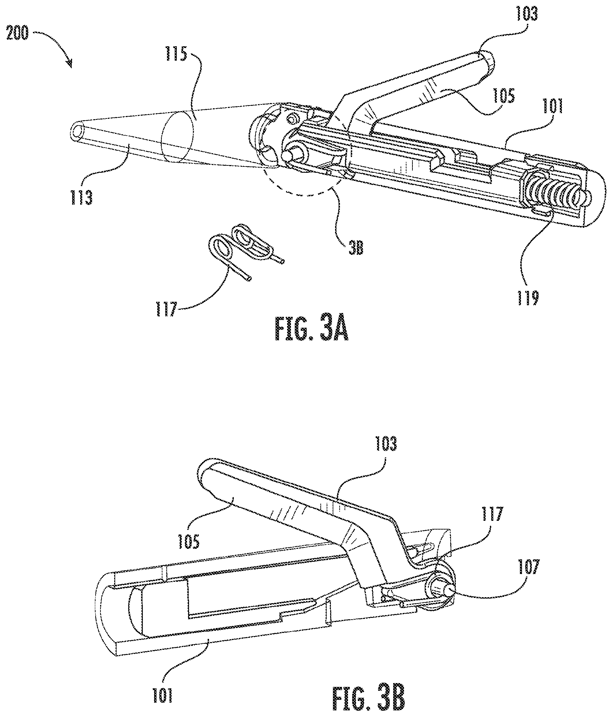

With reference to FIGs. 3A-3B, device 200 is shown with the extension of the cutting surface 105 and blade 103 may be achieved using of stored energy means (e.g., first biasing member 117). For example, stored energy means (e.g., first biasing member 117) can be a torsion or leaf spring and/or a compression spring, which cooperate to ensure smooth movement, can be employed. Blade 103 can be angled, as shown.

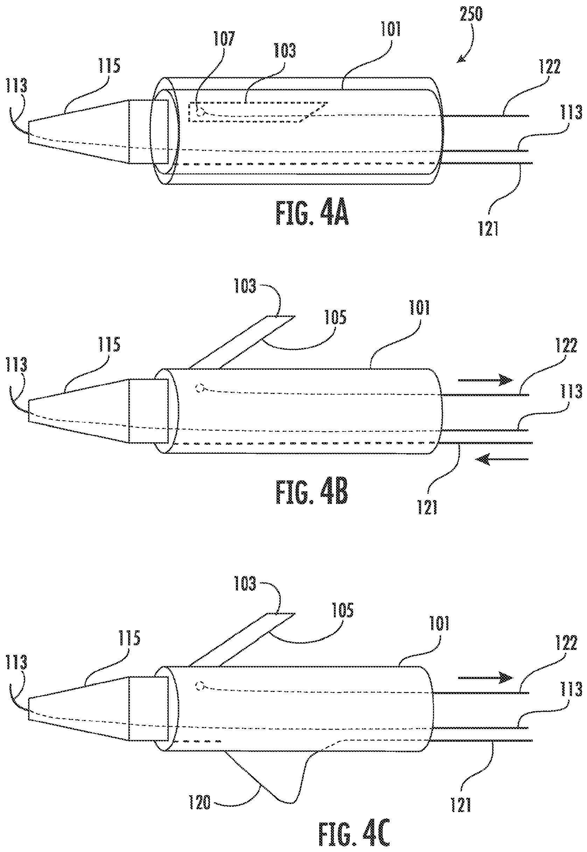

With reference to FIGs. 4A-4C, device 250 is shown in undeployed and deployed configurations, having laterally extending blade 103 in assembly 101, at distal end of flexible catheter (not shown), blade 103 operably coupled to pin 107 and actuator 121 for providing user controlled lateral extension of blade 103 with proximally-facing cutting surface 105 for reverse cutting. Incision assembly 101 in one example is configured to be deployed using a hollow needle/introducer/dilator/guidewire kit for entering a pericardial cavity 54 discussed below. Guidewire 113 feeds thru assembly 101 and atraumatic tip (e.g., introducer 115). Assembly 101 can be configured for over-the-wire (OTW) and introducer 115 can be an offset /rapid exchange guide lumen/channel for advancement over a guidewire 113. Device 250 further comprises a stabilizing member 120 which is independently user controlled by advancing actuating rod 121 distally toward introducer 115 that laterally extends stabilizing member 120 a distance from assembly 101. In one example, catheter 129 is a multi-lumen catheter. In one example, the stabilizing members 120 are wires that come out of a short skive in the multi-lumen catheter, proximal to the incision assembly 101 but instead of returning into the catheter, their distal end is fixed to the outer shell of the distal incision assembly so as to extend the stabilizing members more distally and providing increased support and/or stability. In one example, the wires are drawn filled tube (DFT) wires that are observable within the body of a patient with conventional imaging equipment.

To avoid exerting a forward push force along the multi-lumen catheter, which otherwise might cause undesired deflection thereof, an alternative design can be employed in which each of the stabilizing wires is threaded through a thin-walled tube to create an independent assembly running through a dedicated lumen in the multi-lumen catheter. In such a construct, the tube will be fixed to the multi-lumen catheter at its proximal end while the distal end of wire will be fixed to the tube. When pushed, the wire will extend outward through synced openings in both tube and catheter, whereas the requisite push force will not otherwise exert a force or deflect the multi-lumen catheter 129, as it remains internal to the stabilizing wire and its associated tube assembly, stretching the latter.

In one example, two or more stabilizing members 120 are positioned radially about the assembly 101. In one example, two or more stabilizing members 120 are positioned radially about the assembly 101 about 120 degrees apart. In one example, the two or more stabilizing members 120 are offset longitudinally from the cutting surface 105 to minimize or eliminate pushing the device through the newly cut slit in the pericardium just as it is formed. Stabilizing members 120 can be flexible rods or strip, or inflatable structures, such as balloons that can be inflated with air or liquid (saline). In one example, stabilizing members 120 are inflatable structures, such as balloons that can be inflated with air or liquid (saline) and are used in combination with an RF cutting device. In one example, stabilizing members 120 are inflatable structures, such as balloons that can be inflated with air or liquid (saline) and are used in combination with an RF cutting device to absorb heat from the RF cutting device and reduce or eliminate thermally damage to tissue in proximity to the energized RF cutting device. Thus, in one example, stabilizing members 120 are inflatable structures, such as balloons that can be constructed of thermally conductive and/or thermally absorbing material, inflated with thermally conductive and/or thermally absorbing fluid, or comprise one or more thermally conductive and/or thermally absorbing layers covering at least a portion of the inflatable structure, e.g., proximal to tissue. Examples of thermally conductive or thermally absorbing materials or fluids include thermally conductive paints, thermally conductive epoxy or silicone coatings, or thermal phase change materials (PCMs). In one example, the inflatable structures function as a heat sink during operation of the RF cutting device.

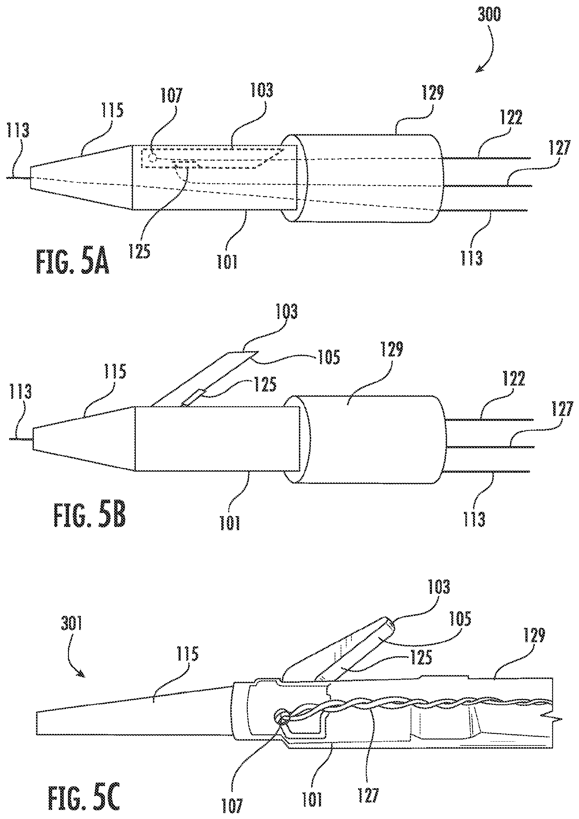

With reference to FIGs. 5A-5C, in one example, device 300 is shown in undeployed and deployed configurations, and has a cutting surface 105 comprising at least one an RF electrode (e.g., cutting tip 125) more proximal to a hinge or pivot pin 107 than cutting surface 105 and blade 103. The operator can choose which cutting surface to use at any point of a procedure when using device 300. For example, if blade 103 is fully laterally extended and pulled into contact with tissue, the RF electrode (e.g., cutting tip 125) would be in position to operate (e.g., where the cutting surface 105 meets the device body). Device 301 of FIG. 5C shows incision assembly 101 coupled to distal end of flexible catheter 129 and to introducer 115.

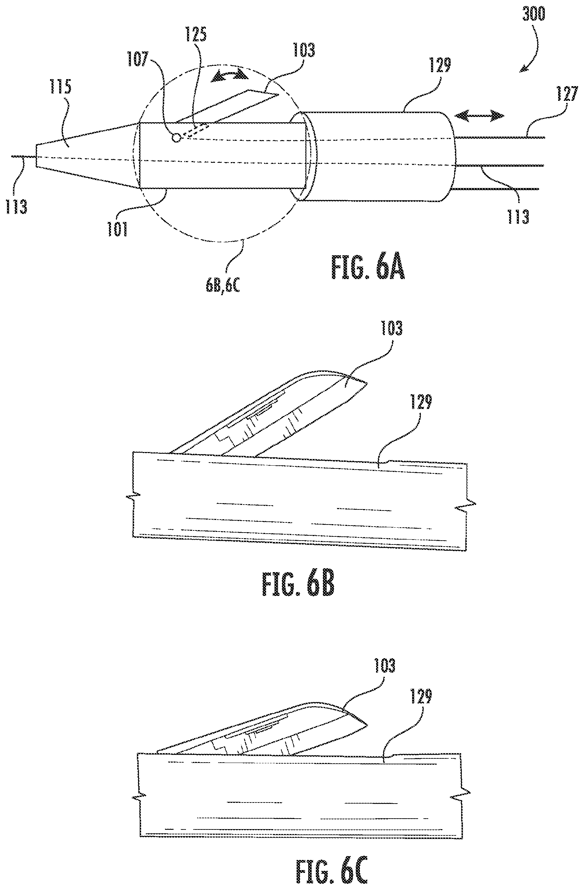

Alternatively, as shown in FIGs. 6A-6C, device 300 could be switched to a blade 103-only mode in which the cutting surface 105 is only partially extended, as depicted in FIG. 6A, such that the blade 103 is in position to operate (e.g., where the cutting surface 105 meets or lies below the device body.) The blade 103 alone or in combination with an electrode (e.g., cutting tip 125) can be user controlled to provide for a scissor-like action, as shown in FIGs. 6B-6C, where blade 103 is actuated proximally and distally from flexible catheter 129 or incision assembly 101. Whether RF or blade 103 is used, the concealed/proximally-facing arrangement of the cutting surface 105 protects surrounding tissues.

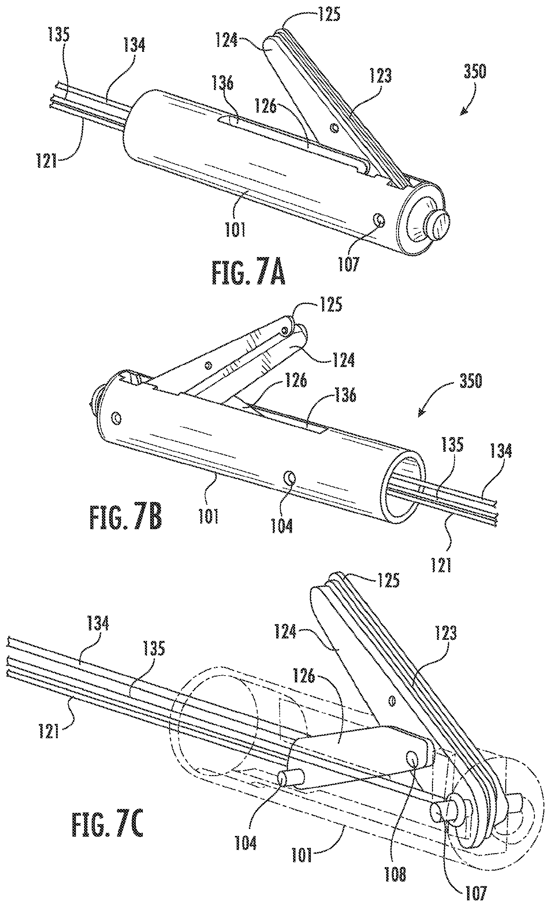

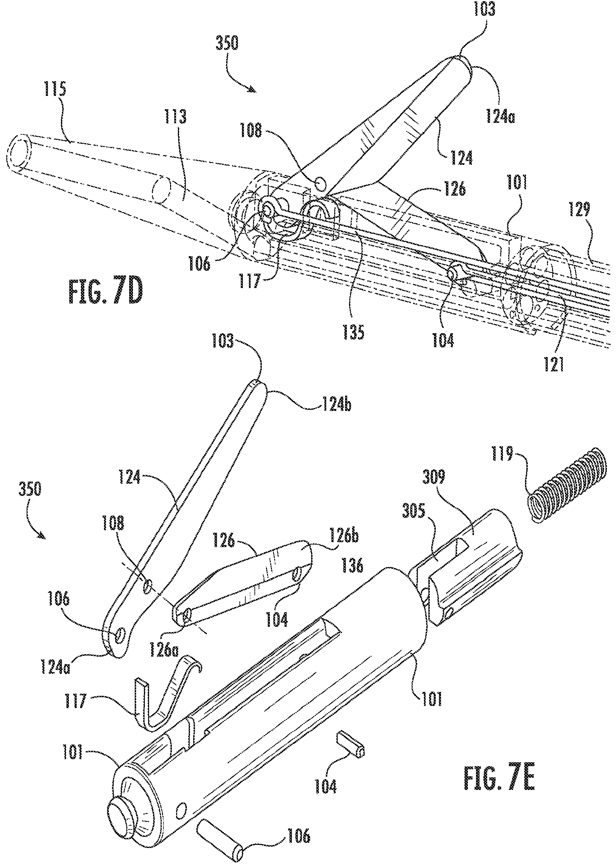

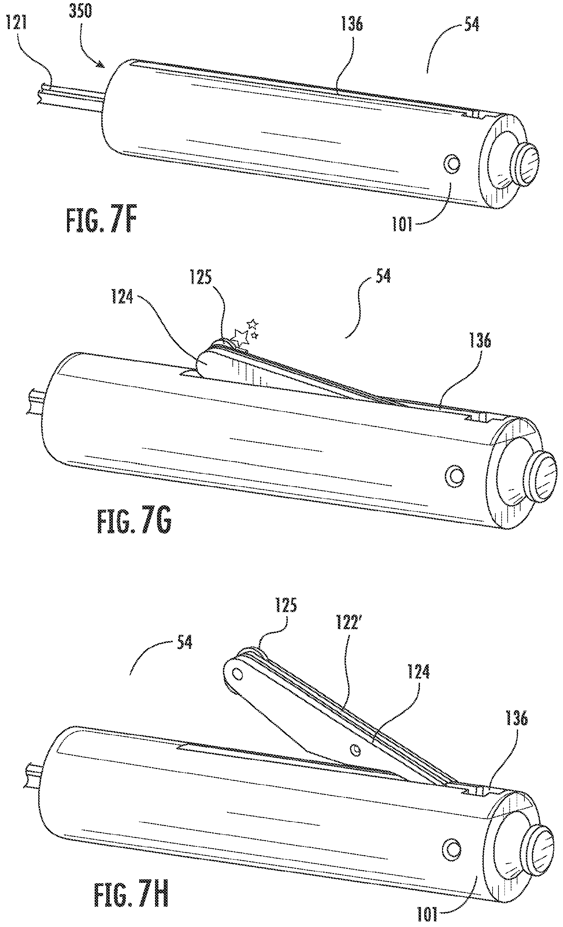

With reference to FIGs. 7A-7F, an exemplary pericardial tissue transection device 350 for creating elongated incisions within a pericardium. Example medical devices, such as device 350 may use a "glide" action and/or a "scissor" action to cut along the wall of the pericardial cavity. The "glide" action maintains the first incision member 124 and the second incision in a fixed position (e.g., angle between the first incision member 124 and the second incision member 126 is fixed) while the incision assembly 101 is being moved along the pericardial. The "scissor" action is an instance in which the first incision member 124 and the second incision member 126 may move relative to one another during the cutting process (e.g., the angle between the first incision member 124 and the second incision member 126 is not fixed and may move to create a scissor force on the tissue).

Device 350 comprises an incision assembly 101 having a longitudinal axis and at least one opening 136. The incision assembly 101 is operably coupled to a distal end of an elongated body 129, such as a flexible and/or steerable catheter or catheter.

Incision assembly 101 comprises a first incision member 124 having a proximal end 124a and a distal end 124b, where the proximal end of the first incision member 124 is pivotably connected to the incision assembly 101. A second incision member 126 having a proximal end 126a and a distal end 126b, with the proximal end of the second incision member 126 pivotably connected to the first incision member 124 is shown. A first biasing member 117 operably coupled to the proximal end 124a of the first incision member 124 and a second biasing member 119 operatively coupled to the distal end 126b of the second incision member 126 provides for reciprocating and/or reversible operation of the two incision members 124, 126 (scissor blades) that can be user controlled. Alternatively, the two incision members 124, 126 may be maintained at fixed positions during the cutting process. The two incision members 124, 126 can be sharpened blades, RF electrodes, or a combination.

Device 350 is adaptably coupled to a controller 1000 described in further detail below. In one example, controller 1000 is operably coupled to the second incision member 126, for example, via an actuator rod 121 that functions to reciprocate the two incision members 124, 126. In one example, the first incision member 124 comprises a first cutting surface arranged in close proximity to a second cutting surface of the second incision member 126 so as to provide a scissoring action and/or glide action sufficient to cut pericardial tissue. As shown, the first incision member 124 and second incision member 126 are axially aligned along a longitudinal axis of the incision assembly 101. The distal end 124b of the first incision member 124 is reversibly adjustable laterally relative to the longitudinal axis of the incision assembly 101 between a range of angles so as to provide a scissoring action and/or glide action sufficient to cut pericardial tissue.

The proximal end 124a of the first incision member 124 is pivotably connected to the incision assembly 101 at a first connection point 106, and the distal end 126b of the second incision member 126 is pivotably connected to the incision assembly 101 at a second connection point 104. The proximal end of the second incision member 126 is pivotably connected to the first incision member 124 at third connection point 108. As shown, the third connection point 108 is positioned between the first connection point 106 and the distal end 124b of the first incision member 124, e.g., point 108 is spatially separated from pivot point 106.

A first biasing member 117 is operatively coupled to the incision assembly 101 and to the proximate end of the first incision member 124. The first biasing member 117 exerts a rotary force or torque to the first incision member 124, laterally projecting the distal end of the first incision member 124 through an opening 136 of the incision assembly 101. The second biasing member 119 exerts a pushing or spring force to the distal end 126b of the second incision member 126, pushing the distal end 126b of the second incision member 126 longitudinally towards the first biasing member 117.

In this configuration, distal end 126b of the second incision member 126 is reversibly adjustable parallel to the longitudinal axis of the incision assembly 101. With distal end 126b of the second incision member 126 operably connected to actuator rod 121, translation of distal end 126b controlled by the controller via actuator rod 121, provides for reciprocation of the two incision members 124, 126 against one or both of the biasing forces of biasing members 117, 119. The first biasing member 117 can be at least one of a torsion spring and a compression spring. The second biasing member 119 can be a spring.

As shown in FIG. 7E, distal end 126b of the second incision member 126 is operably connected to a yoke 309, where the yoke comprises a yoke lumen 305 that receives the distal end 126b of the second incision member 126. In one example, at least a portion of the actuator rod 121 is positioned within the yoke lumen 305.

Transection device 350 can further comprise a sheath (not shown), where the sheath is slidably traversable over an outer diameter of the incision assembly 101. Thus, with a sheath, a first configuration, where the sheath is located over the at least one opening 136 of the incision assembly 101 is provided, where in the first configuration the sheath counteracts the bias of the first biasing member 117 and prevents lateral projection of the first incision member 124 from the incision assembly 101.

The transection device of any one of the previous claims, further comprising a second configuration, where the at least one opening of the incision assembly 101 is uncovered, laterally projecting the first incision member 124 through the at least one opening 136 of the incision assembly 101.

In one example, the transection device is operated by manipulating the controller so as to exert a pulling force to the actuator rod 121, counteracting the force of the second biasing member 119 and traversing the distal end 126b of the second incision member 126 parallel to the longitudinal axis of the incision assembly 101.

In one example, the distal end 124b of the first incision member 124 comprises an electrically conductive tip 125 operably coupled to an independent source of radiofrequency energy or electrical current. In this manner, the tip 125 can be operated independently of first incision member 124, as the first incision member 124 includes a non-conductive element 123 (e.g., an insulating surface) electrically isolating tip 125. In this configuration, the opposite sides of the first incision member 124 are independently operably coupled to independent sources of radiofrequency energy or electrical current via wires 134, 135. In one example, the controller is configured to independently operate the source of radiofrequency energy or electrical current to the first incision member 124 and the second incision member 126 and the conductive tip 125.

The transection device of any one of the previous claims, where the first incision member 124 comprises an electrode.

The transection device 350 can have one or more stabilizing members 120 located adjacent to the at least one opening of the incision assembly 101. In one example, the one or more stabilizing members 120 reversibly projects laterally about 120 degrees radially apart about the incision assembly 101. The one or more stabilizing members 120 can be a wire, loop, or shape-memory metal, or one or more inflatable structures. In one example, the one or more stabilizing members 120 prevents rotation of the incision assembly 101 around the longitudinal axis.

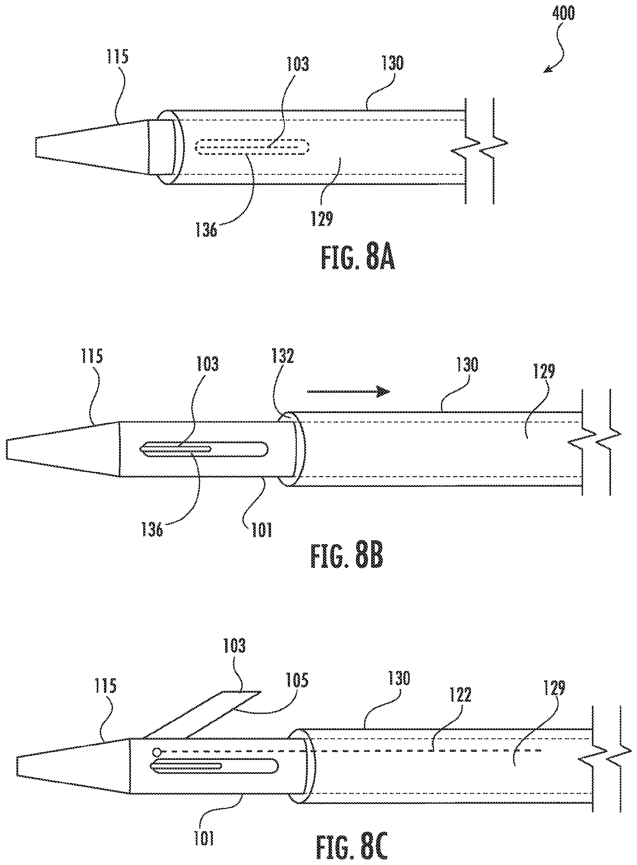

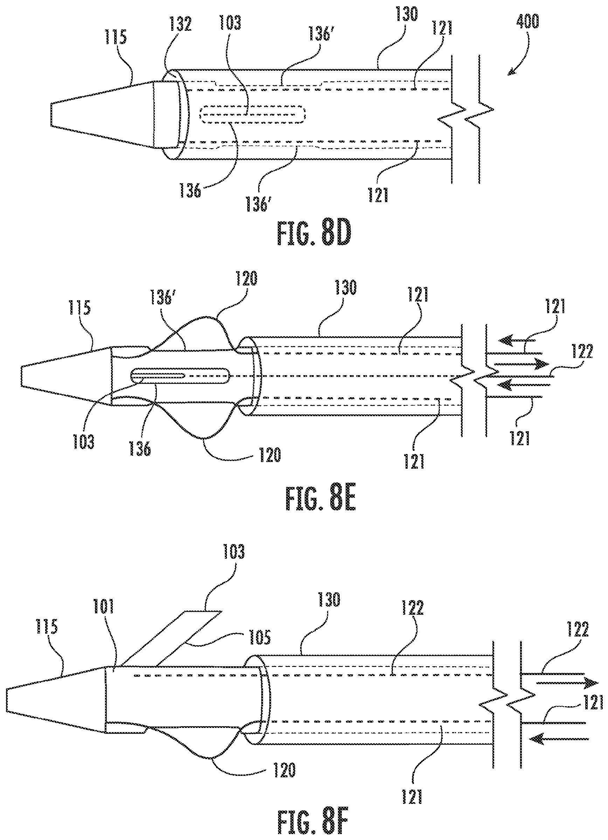

With reference to FIGs. 8A-8F, an exemplary transection device 400 is depicted in undeployed and deployed configurations, using a sheath 130 over the flexible catheter 129 and the incision assembly 101. Distal end 132 of sheath 130 can be drawn back from assembly 101 allowing incision device to laterally extend from assembly opening 136. As shown, blade 103 is extended thru assembly opening 136 with the traversal of distal end 132 of sheath 130. With reference to FIGs. 8D-8F, device 400 is depicted with stabilizing members 120 that extend laterally and concurrently from additional assembly openings 136' with incision device upon retracting sheath 130, or independently by user controlled deployment via controller/actuator rods 121. Alternatively, incision device can be coupled to an actuator and/or controller for user deployment after sheath 130 retraction and retraction of cutting surface after cutting, or a passive biasing force can trigger release of the incision device upon sheath 130 retraction and after cutting, the sheath can be advanced to engage and cover a portion of the cutting surface for subsequent removal of the device.

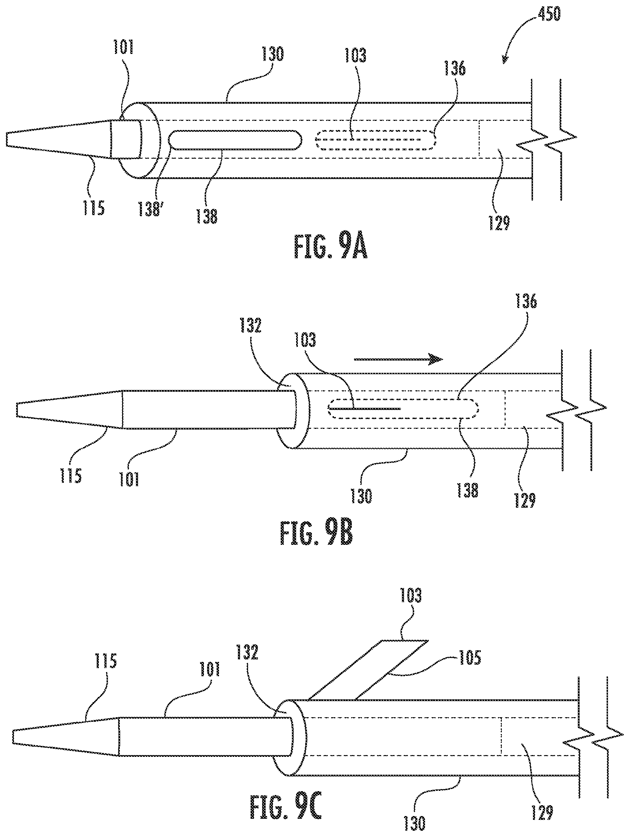

With reference to FIGs. 9A-9C an exemplary transection device 450 is depicted in undeployed and deployed configurations, using a sheath 130 over the flexible catheter 129 and the incision assembly 101. Sheath 130 includes at least one sheath opening 138 that can be drawn back from assembly 101 and to align sheath opening 138 with assembly opening 136 allowing incision device to laterally extend therefrom. Stabilizing members 120 as disclosed above, that extend laterally and concurrently from additional assembly openings 136' with incision device upon retracting sheath 130, or independently by user controlled deployment via controller rods/actuator can be used. In one example, incision device can be coupled to an actuator 121 for user deployment after sheath 130 retraction and retraction of cutting surface after cutting, the sheath can be advanced to engage and cover a portion of the cutting surface for subsequent removal of the device. Alternatively, a passive biasing force can trigger release of the incision device upon sheath 130 retraction and after cutting, distal end 138'of sheath opening 138 is used to engage with the back side of the incision device with further withdrawal of sheath 130 and for retraction of the incision device for removal after cutting. In one example, at least a portion of the sheath opening 138 is radiopaque.

In one example, sheath opening 138 is partially overlapping assembly opening 136 with the incision device in a retracted configuration. Such overlapping provides for minimizing the withdrawal distance needed to actuate lateral extension of incision device and/or retraction of the incision device by distal end 138' of sheath opening 138.

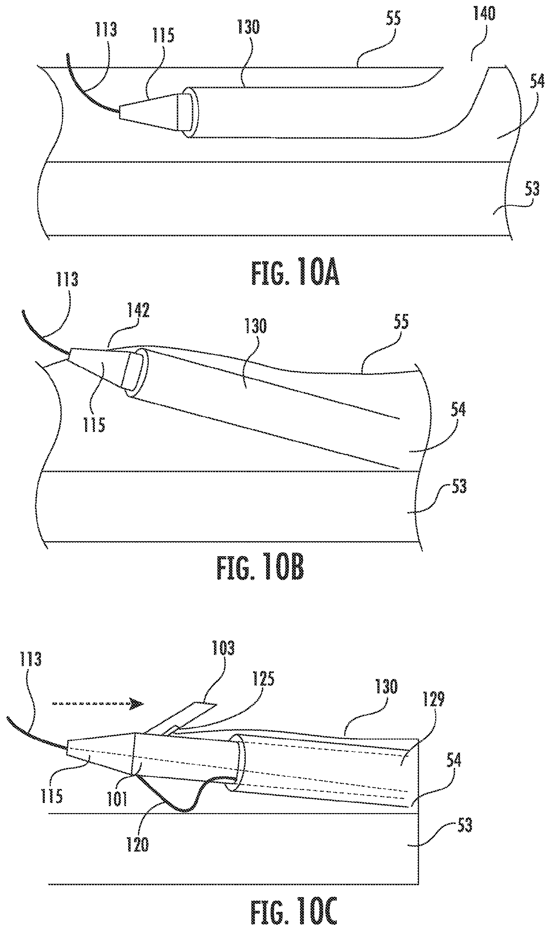

With reference to FIGs. 10A-10C the presently disclosed transection devices can be advanced through a subject's pericardial cavity 54 in undeployed configuration with the cutting surface 105 retracted against/into the housing or assembly 101 of the device, e.g., like a folding pocket knife. However, unlike a folding pocket knife that can pivot the blade 180 degrees, the cutting surface 105 of the presently disclosed device is configured to extend laterally from the housing or assembly 101 or catheter (and collinearly) at an angle of about 90 degrees or less in its deployed configuration. Thus, in one example, when fully extended, the cutting surface 105 of the presently disclosed device forms an acute angle with the body of the device and/or the flexible catheter 129 used to introduce the device into the pericardial cavity 54. Thus, for example, when the cutting surface 105 of the presently disclosed transection device is presented, it forms an angle of about 90 degrees or less (e.g., less than 90, 80, 70, 60, 50, 40 degrees or less) with the flexible catheter 129.

With reference to FIG. 10A, a method of using transection device 400 is shown were flexible catheter 129 with incision assembly 101 and introducer 115 is advanced (e.g., over a guidewire 113) through a punctured entry hole or access point 140 thru the pericardium and into the pericardial cavity 54, within/under the pericardium and advanced to a cut-start site or target site 142 (which is at the distal end of the path to be cut) in its undeployed configuration. In one example, as shown in FIGs. 10B, 10C, guidewire 113 is caused to exit the pericardial cavity 54, the cutting surface 105 or RF electrode is then extended and transection device 400 is retracted along the cutting path - i.e. the cutting is performed while pulling the device back toward the punctured entry hole, for example, along guidewire 113 with transection device 400 in its deployed configuration. Thus, pre-cutting advancement of the device is employed by advancing the distal end of the device out of the pericardium at the cut-start site 142, e.g., using a guidewire 113, prior to extending the cutting surface 105 outside of the pericardium.

The above method has the cutting surface 105 return to the outside of the pericardium at the cut-start site 142 such that the cutting surface 105 can traverse the pericardial tissue to be cut.

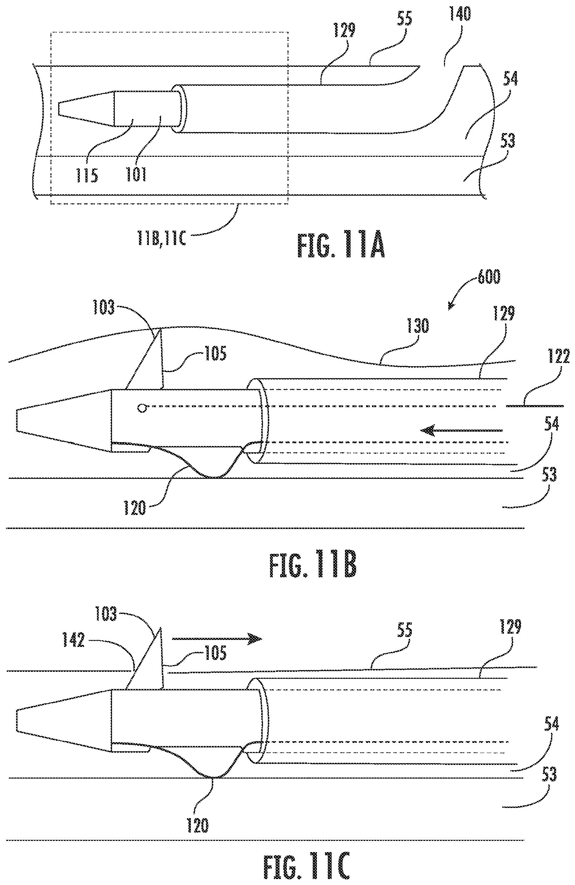

Alternatively, with reference to FIGs. 11A-11C transection device 600 uses a portion of blade 103 having a pointed tip to exit or "puncture out" of the pericardial cavity 54 when the blade 103 and cutting surface 105 is laterally extended while still in the pericardial cavity 54. The transection device 600 is pulled, and the pointed tip engages the inner surface of the pericardium and pierces through it allowing the cutting surface 105 to present to the pericardium tissue, e.g., the parietal layer 55. In one example, pointed tip blade can also be configured to extend bluntly so as to keep a portion of blade above the pericardium and minimize or eliminate loss of position or slip during cutting (e.g., reverse cutting). In addition, one or more extensions from tip of blade can serve as a radiopaque marker indicating 'bunching up' of tissue, for example, due to excessive pull before a through-cut is achieved. In one example, the back silhouette of the blade may be selectively insulated (as it may be activated to achieve the exit puncture) to avoid ablation damage to neighboring anatomy.

Stabilizing member 120 can be concurrently or subsequently laterally extended, for example, to assist with the puncturing of the pericardium by pointed tip of blade 103 or alternatively or in combination, the reverse cutting of the pericardium. The cutting may be achieved by simply slicing the pericardium by pulling of the device while the cutting surface 105 remains extended similar to a letter opener.

Alternatively or additionally, the cutting may be achieved/facilitated/augmented by retracting and re-extending the cutting surface 105 similar to scissors. The body of the device may have a complementary cutting surface (not shown) as previously described, to facilitate the scissoring effect.

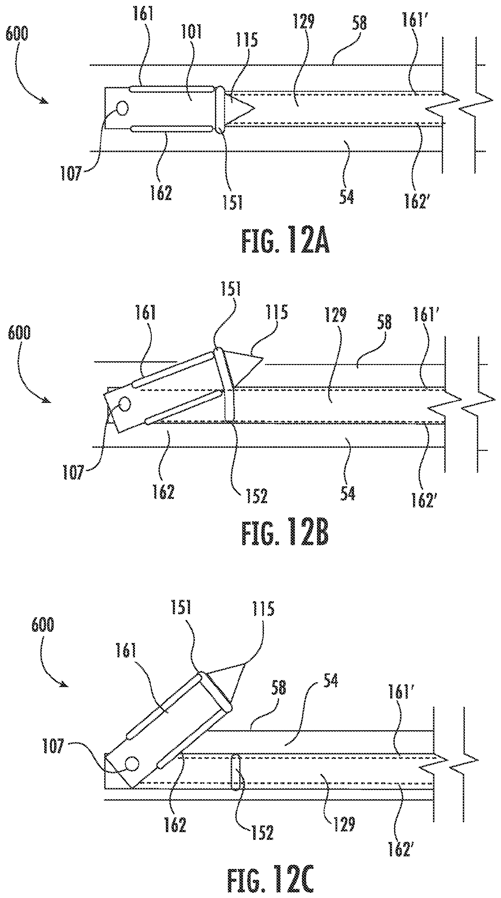

FIGs. 12A, 12B, 12C, depict an exemplary transection device 600 with a jack-knife configuration, capable of puncturing out of the parietal cavity 54 using RF or current and also configured to arrange a set of cutting electrodes and/or sharpened edges for reverse cutting of at least the parietal layer 55. Transection device 600 has an elongated body 129 comprising a distal end. An incision assembly 101 can be coupled to the distal end of the catheter. A first radiopaque marker 152 can be positioned about the elongated body 129. A second radiopaque marker can be positioned about the incision assembly 101. Transection device 600 is configured to transition between a first configuration (FIG. 12A) where the first radiopaque marker 152 and the second radiopaque marker 151 are in proximity to each other, and a second configuration (FIGs. 12B, 12C) where the first radiopaque marker 152 and the second radiopaque marker 151 are in spatially separated from each other. As shown, transection device 600 has incision assembly 101 pivotably coupled to distal end of an elongated body 129 (catheter or catheter) at pivot pin 107. Incision assembly 101 of transection device 600 includes incision member 162, tapered atraumatic tip (e.g., introducer 115), and optional puncturing member 161 to punch out and/or cut the parietal layer 55. In one example, incision member 162 is a sharpened edge and/or a RF electrode electrically coupled to controller via conductive wire 162'. Optional puncturing member 161 can be an RF electrode electrically coupled to controller via conductive wire 161' that provides independently controlled current or RF energy between incision member 162 and puncturing member 161. Transection device 600 as shown comprises a plurality of spatially separable radiopaque markers 151, 152 to provide for visualization of punching out of the pericardial cavity 54 as well as the spatial relationship of the incision member 162 and the elongated body 129 during a procedure. Optionally, elongated body 129 can include a third incision member (not shown) in reversible proximity to incision member 162 for additional cutting effect. Third incision member can be independently controlled with current or RF energy between one or both of incision member 162 and puncturing member 161.

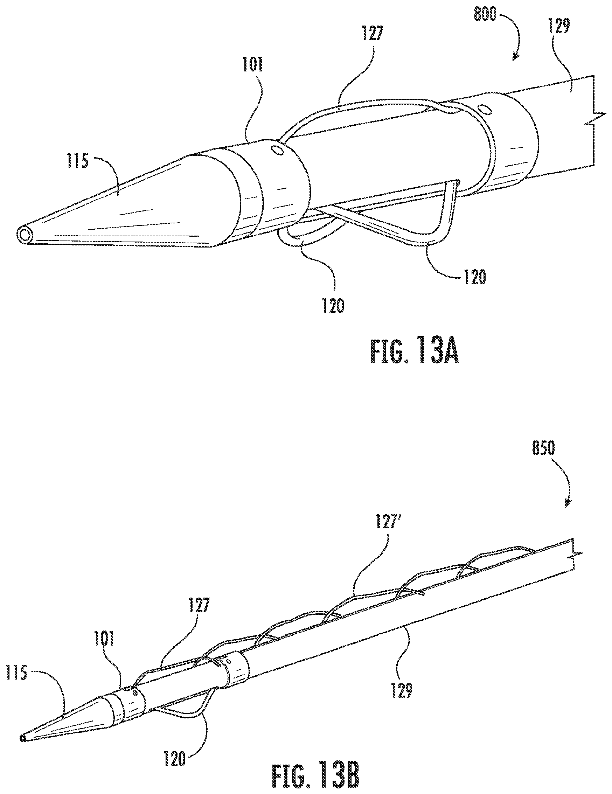

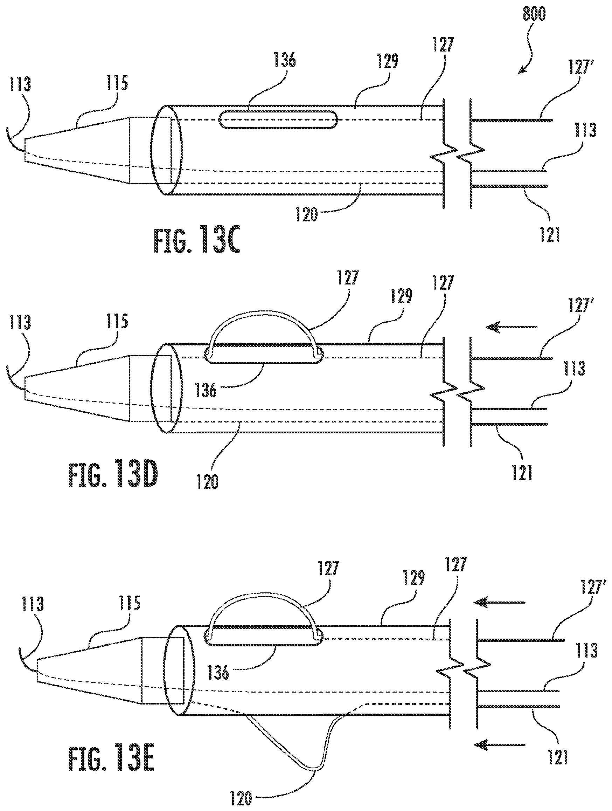

With reference to FIGs. 13A, and 13C-13E, an exemplary device 800 is depicted employing an RF cutting surface 127 that is user-actuated at distal end of flexible catheter 129. RF cutting surface 127 can be arranged in assembly 101 with suitable electrical feed and insulation for use. Introducer 115 is distally arranged from assembly 101 and can be threaded with a guidewire as previously discussed. In one example, a single arc of wire is used in the same position in which the blade 103 is otherwise located or positioned about the device and can be laterally extended thru assembly opening 136 by a controller. In one example, the wire(s) are configured to carry the current, independently, is electrically coupled solely to the blade or a puncturing member insulated from blade, while assembly 101 and/or catheter 129 acts as insulator. Stabilizing members 120 can be concurrently or subsequently laterally extended as discussed above.

With reference to FIG. 13B device 850 is shown having multiple arcs of wire 127' along flexible catheter 129. In one example, the multiple arcs of wire 127' along flexible catheter 129 are substantially colinear with each other and the longitudinal axis of the device 850, as shown. The multiple arcs of wire 127' along flexible catheter 129, in one example, provide for single segment cutting of predetermined incision length. The multiple arcs of wire 127' along flexible catheter 129, in one example, provide for selective independent control over which arc(s) are active at which time and/or their respective, independent current load. The multiple arcs of wire 127' along flexible catheter 129, in one example, provides selective application of energy along entire path of arcs concurrently, sequentially or randomly. The multiple arcs of wire 127' along flexible catheter 129, in one example, provide greater consistency of energy along the overall path (whereas one single wire may apply different amounts of energy at different regions of the wire). The multiple arcs of wire 127' along flexible catheter 129, in one example, provide for multi segment cutting, e.g., cutting one segment at a time, with arc positions pre-arranged to perform a contiguous cut, a perforation, etc. Stabilizing members 120 can be concurrently or subsequently laterally extended as discussed above for device 600.

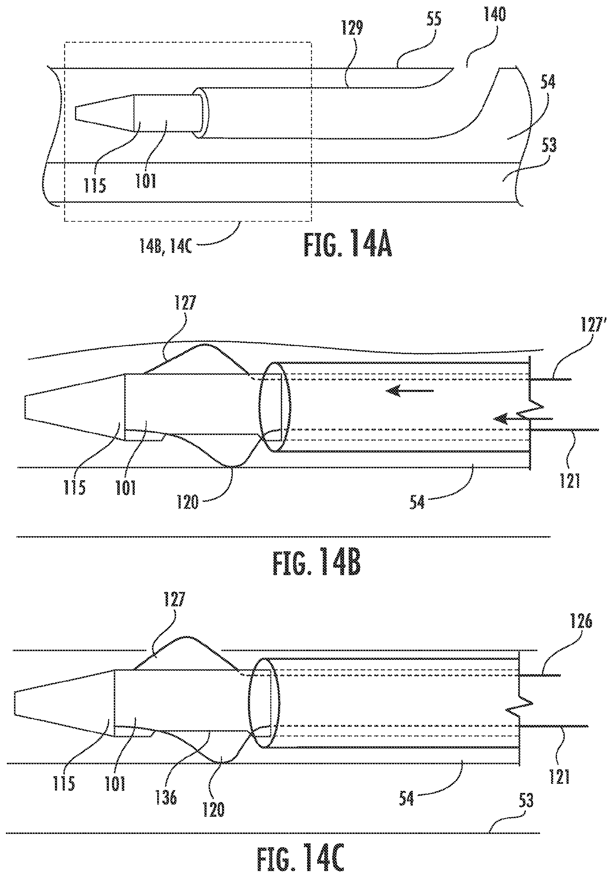

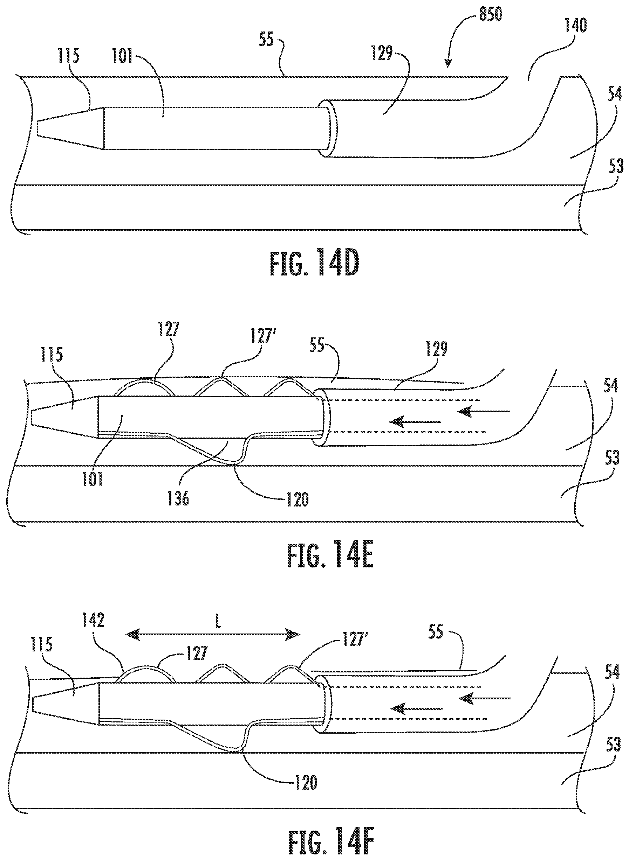

With reference to FIGs. 14A-14C, a method of using device 800 is shown, where entry into the pericardial cavity 54 is made at access point 140 and device 800 is advanced to cut site in undeployed configuration where cutting surface 127 is laterally extended from opening 136 of incision assembly 101 in its deployed configuration and supplied with current or RF sufficient to cut thru the parietal layer 55. Stabilizing members 120 can be concurrently (to assist with breaking thru pericardium using current or RF) or subsequently laterally extended as discussed above.

With reference to FIGs. 14D-14F, a method of using device 850 is shown, where entry into the pericardial cavity 54 is made at access point 140 and device 850 is advanced to cut site or cut length(s) are desired, where plurality of cutting surfaces (e.g., wire(s) 127, 127') are laterally extended from opening 136 of incision assembly 101 so as to provide an extended cut length L that can be continuous or segmented/perforated while device 850 remains essentially stationary in pericardial cavity 54. Stabilizing members 120 can be concurrently deployed with cutting surfaces 127, 127' (to assist with breaking thru pericardium) or subsequently laterally extended as discussed above.

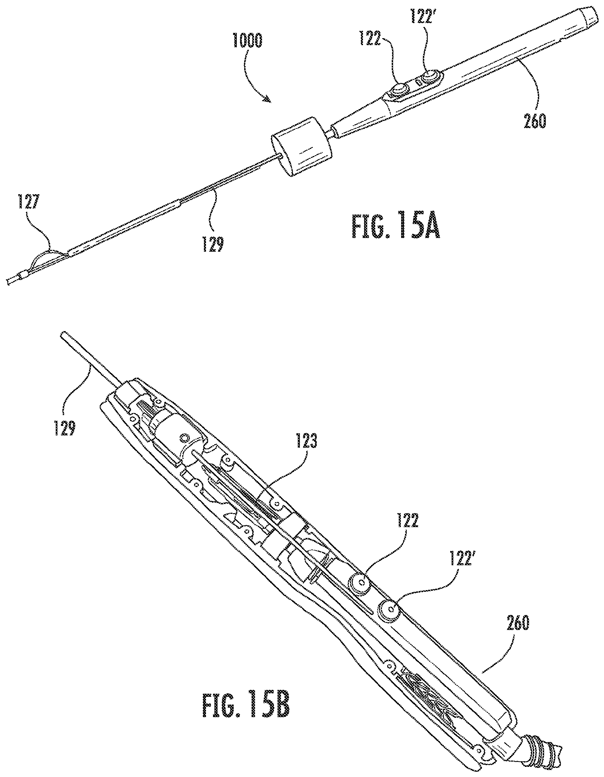

With reference to FIGs 15A-15B, exemplary controller 1000 is shown having handle 260, actuating buttons 122, 122' for operably coupling with the incision device, for example actuator rod 121, retracting sheath 130, extending stabilizing members 120 via rods 121, etc. In one example, the controller 1000 facilitates various potential operations of the incision assembly 101 components, including biasing/clamping the distal blade open and closed, which can be achieved by an appropriate mechanism configured to pull/push a rod. In one example, there is a mechanism used to release/retrieve balloons/nitinol components that function to stabilize and apply counterpressure for the incision assembly 101 and its components, including the cutting surface. Controller 1000 may comprise one or more buttons 122, 122' used to operate and control the electrosurgical features of the device, e.g., current and RF.

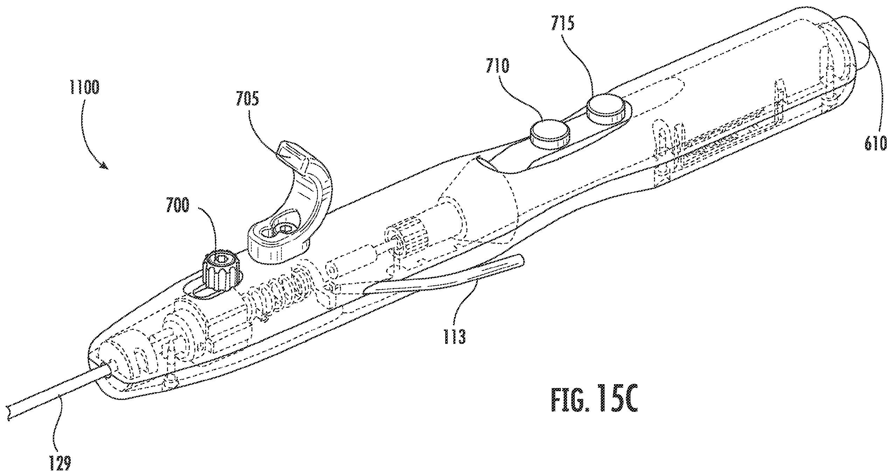

With reference to FIG. 15C, any of the previously disclosed transection devices can be manipulated and/or controlled from outside the subject using alternative controller 1100, which may be a handle. The controller 1100 may have multiple actuating knobs 700, 705, 610 and actuating buttons 710, 715 for controlling the catheter 129 and the various components of the transection device. Knob 700 may be configured to rotate the flexible catheter 129 in response to orientation information derived from fluoroscopy or other visualization means. Knob 705 may be engaged to activate one or more components on the medical device (e.g., actuating the stabilizing member(s)). Similarly, the actuating buttons 710, 715 may be engaged to activate various components of the catheter (e.g., the device may use RF electrode cutting as well and the actuating buttons 710, 715 may be used to supply current or RF) and guidewire control. Various other controllers are envisioned that allow for the aforementioned transection devices to be deployed and manipulated.

In one example, the presently disclosed devices are configured for introduction to the pericardial cavity 54 and for cutting tissue layers generally disposed adjacent to and including adipose tissue within and outside the pericardial cavity 54 and superficial to the visceral layer 53 of the pericardium 60.

In one example, to provide orientational stability of the cutting surface to that of the parietal layer, an OTW introduction is employed for any of the previously disclosed devices, for example, whether through a dedicated lumen in catheter cross-section or 'Rapid Exchange' style catheter, or off-center attached cannula, or deflect-resistant catheter. In one example, the delivering catheter comprises radiopaque material randomly distributed or arranged in a pattern for visualization using conventional visualization techniques during use.

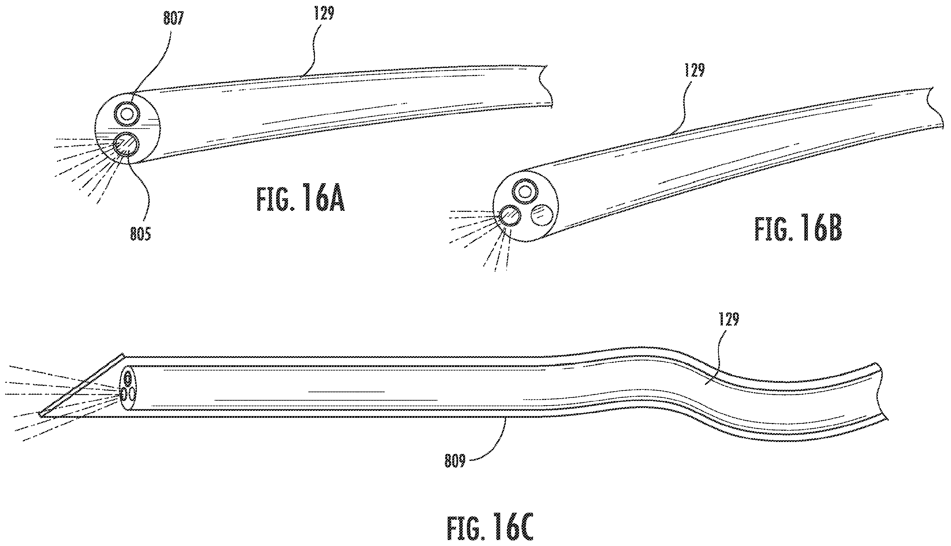

Current ECHO/fluoroscopy may not provide the required visualization for certain access applications of the presently disclosed transection devices, for example, gaining guidewire access to the pericardial cavity consistently and repeatedly may be desired. Thus, in one example, the catheter device 129 coupled to the presently disclosed transection devices comprises direct visualization, as shown in FIG. 16 allowing the user to watch real-time the advancement of any of the presently disclosed transection devices traverse through various tissue layers until the desired location is reached. Changes in tissue layers that may not be visible under ECHO/fluoroscopy may be easily distinguishable under direct visualization such as tissue/bloodstream (vessel access), myocardium/pericardium (pericardial cavity access), myocardium/pericardium (outside pericardium), among other anatomical features.

Thus, in one example, the presently disclosed devices discussed above further comprise an optical channel in the catheter to accommodate a lens coupled to a fiber optic cable, optionally with a light source, e.g., an LED. In one example, the presently disclosed method further comprises obtaining visual information during accessing, traversal of the pericardial cavity, exiting and/or cutting, for example, using an optical channel in the catheter to accommodate a lens coupled to a fiber optic cable, optionally with a light source, e.g., an LED.

As shown in FIG. 16, catheter 129, shown without a pericardial transection device for clarity, comprises a fiber optic channel and lens 807 adjacent a fiber optic channel 805 to provide light and to provide an analog or digital image in a catheter 129, which may also have a sheath 809.

In one example, a puncture to deliver a guidewire into the pericardial cavity 54 is performed through heart tissue in a transvascular approach. When a transvascular approach through the RAA, IVC, or SVC is employed, a closure device (e.g., occluder) may be subsequently introduced for hemostasis at the conclusion of the procedure. In one example, the closure device includes outward or radially directed splines deployed in an expanded configuration. When the guide catheter is removed, the splines or radial members of the closure device contract inwardly towards the unstressed state of the transection device in order to close, occlude, and/or seal the opening. Thus, the closure device is designed such that a pericardial cutting device can pass through and into the pericardial space.

The following exemplary occlusion descriptions relate to a transvascular approach through the RAA, IVC, or SVC using one of the aforementioned transection devices. In one example, a hollow needle delivers a wire into the pericardial space through heart tissue. A closure or occlusion device can be introduced for hemostasis at the conclusion of the procedure if the outer diameter of the transection device is greater than 7 Fr. The closure or occlusion device in one example includes outward or radially directed splines deployed in an expanded configuration. When the guide catheter is removed, the splines or radial members of the closure device contract inwardly towards the unstressed state of the transection device in order to close and seal the opening. The closure device is designed such that a pericardial cutting device can pass through and into the pericardial space.

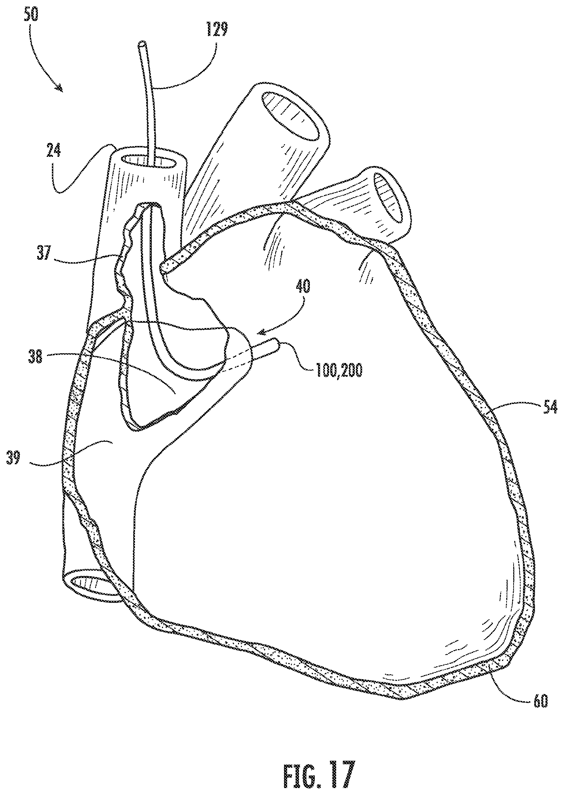

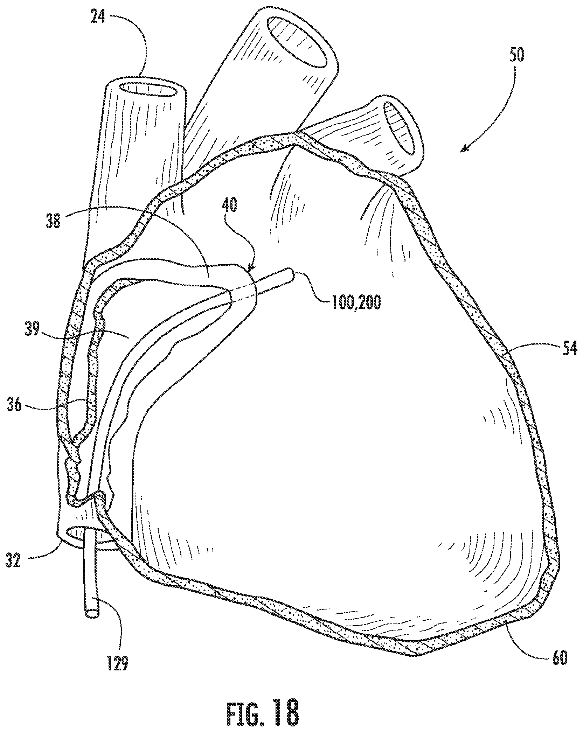

FIGs. 17, 18 shows exemplary intravascular approaches for delivering the transection devices of the present disclosure to the pericardial cavity 54. Thus, FIG. 17 depicts heart 50 viewed in isolation from the body, with the pericardium 60 or pericardial sac encasing the cardiac muscle (i.e., epicardium, myocardium, and endocardium). The small space which is present between the heart muscle and pericardium 60 represents the pericardial cavity 54.

The presently disclosed transection devices can be presented to the pericardial cavity 54. In one example via the right atrial appendage 38 (RAA), which is a suitable site for entry into the pericardial cavity 54, is used. Right atrial appendage 38 lies tangential to and between pericardium 60 and the epicardium/epicardial adipose tissue 57. In one example, any of the presently disclosed devices can be guided into right atrial appendage 38 via right atrium 39 so as to be positioned substantially in parallel with the wall of pericardium 60 such that the wall of right atrial appendage 38 can be pierced by any of the presently disclosed devices substantially without risk of damaging the epicardium or other heart tissue. Other access routes to the pericardial cavity can be used, for example, direct "puncture out" of SVC or IVC/coronary sinus (CS) and a "puncture into" the pericardium. A range of techniques can be used to exit these cardiac and vascular structures including the use of percutaneous endovascular approaches that utilize catheters, needles, and/or radiofrequency energy pass from within these structures to the pericardial space.

In some examples, right atrial appendage 38 is accessed via conventional vena cava routes. FIG. 17 illustrates entry of any of the presently disclosed devices into right atrium 39 via the superior vena cava 24 (SVC). A cut-away 37 shows passage of any of the presently disclosed devices through superior vena cava 24, right atrium 39, and right atrial appendage 38. A distal tip of catheter 129 is shown exiting right atrium 39 at apex 40.

FIG. 18 illustrates an alternative entry of any of the previously disclosed devices into right atrium 39 via the inferior vena cava 32 (IVC). A cut-away 36 shows passage of catheter 129 through inferior vena cava 32, right atrium 39, and right atrial appendage 38. A distal tip of catheter 129 is shown exiting right atrium 39 at apex 40.

Thus, by way of example, a method of reducing pericardial restraint of a subject in need thereof using any of the presently disclosed devices is provided by the following steps. Any of the presently disclosed devices is maneuvered through one of the vena cava 24, 32 to right atrium 39. Once inside right atrium 39, any of the presently disclosed devices is passed into the right atrial appendage 38. The wall of right atrial appendage 38 is pierced at apex 40, and the catheter is advanced into the pericardial cavity 54. Other transvascular-right heart routes to the pericardial cavity 54 are envisaged. Furthermore, left atrial appendage, coronary sinus, and right ventricle pathways are envisaged for transvascular access to the pericardial cavity 54.

Note that the wall of the right atrial appendage may be pierced with any of the presently disclosed devices itself, or with an instrument (e.g., guidewire) passed through a lumen of the any of the presently disclosed devices, e.g., over the wire. Further, any of the previously disclosed devices may be passed into the pericardial space through the opening in the wall of the atrial appendage, or an instrument passed through the lumen of any of the presently disclosed devices may be presented into the pericardial cavity 54. These details will depend on the procedure being performed and on the type of the previously disclosed device being employed.

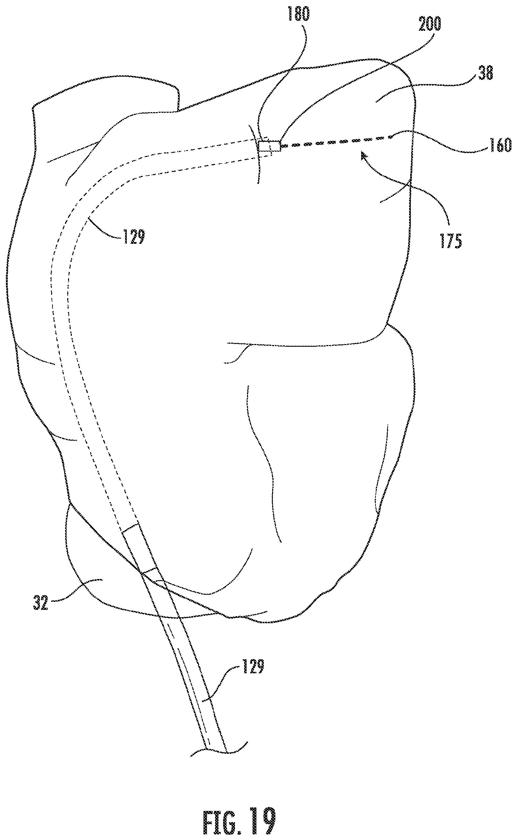

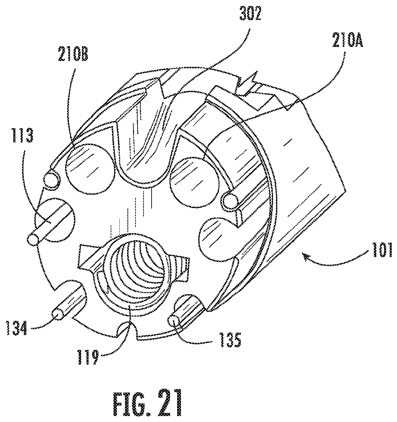

As shown in FIG. 19, any of the presently disclosed devices can be used to create a cut path of a length in a pericardium, e.g., in a parietal layer 55. Thus, a catheter 129, e.g., a steerable catheter can be employed, extending through the IVC, through the RA, and into the RAA, for example, and then into the pericardial cavity 54. The catheter 129 may have one or more steerable segments guiding any of the presently disclosed devices, with a radius of curvature of between about 1 inch and about 5 inches, with an arc length of between about 90° and about 180°. 1 inch = 2.54 cm. As exemplary shown in FIG. 21, any of the presently disclosed devices can be positioned in the pericardial cavity 54 and can begin a cut path 175 at a start point 160 and ends at endpoint 180 of a length. At least a portion of the parietal layer 55 of the serous pericardium, and the fibrous pericardium 56, and pericardial adipose tissue 59 are separated along cut path 175. The one or more incisions along the lengths, in a heart with a dysfunction treatable with the present method, cause the pericardium to separate about the cut line of the incision or cut path 175, e.g., about the circumference of the heart, without the removal of pericardial tissue and with a reduction in pericardial restraint. One or more cut paths 175 can be made, and different cut paths, of various lengths can be used to reduce pericardial restraint. In one example, the cut path 175 and its length is pre-operatively determined. Other cut paths and lengths can be used.

In one example, the presently disclosed device further comprises at least one nerve detection device. In one example, the at least one nerve detection device is located on the flexible catheter 129. In one example, the at least one nerve detection device is located adjacent the incision assembly 101. In one example, the at least one nerve detection device is located on the incision blade 103.

Any one of the presently disclosed devices can further comprises at least one nerve stimulation device. In one example, the at least one nerve stimulation device is located on the flexible catheter 129. In one example, the at least one nerve stimulation device is located adjacent the incision assembly 101. In one example, the at least one nerve stimulation device is located adjacent the blade 103.

The presently disclosed pericardial transection devices and/or catheter and/or sheath can be configured such that the total outer diameter (O.D.) introduced to the pericardial cavity is between about 6 Fr (2mm) and about 30 Fr (10mm). In one example, presently disclosed pericardial transection devices and/or catheter and/or sheath can be configured such that the total outer diameter (O.D.) introduced to the pericardial cavity is between about 6 Fr (2mm) and about 20 Fr (6.67mm). The pericardial transection device and/or catheter and/or sheath can be configured such that the total outer diameter (O.D.) introduced to the pericardial cavity is between about 6 Fr (2mm) and about 15 Fr (5mm). In one example, presently disclosed pericardial transection devices and/or catheter and/or sheath can be configured such that the total outer diameter (O.D.) introduced to the pericardial cavity is between about 6 Fr (2mm) and about 12 Fr (4mm). In one example, presently disclosed pericardial transection devices and/or catheter and/or sheath can be configured such that the total outer diameter (O.D.) introduced to the pericardial cavity is approximately 10 Fr (3.33 mm).

Any of the above disclosed devices are configured for aseptic manufacturing and packaging, ethylene oxide or autoclave sterilization, or high energy sterilization, e.g., e-beam, gamma, UV.

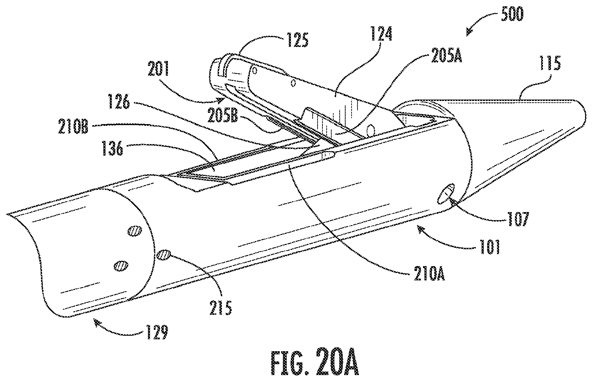

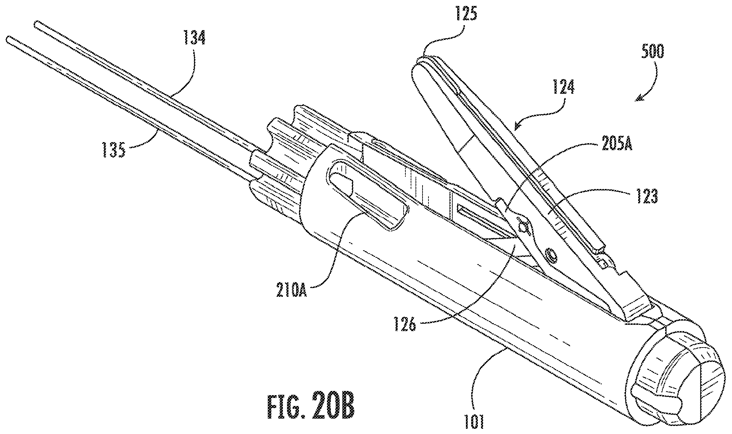

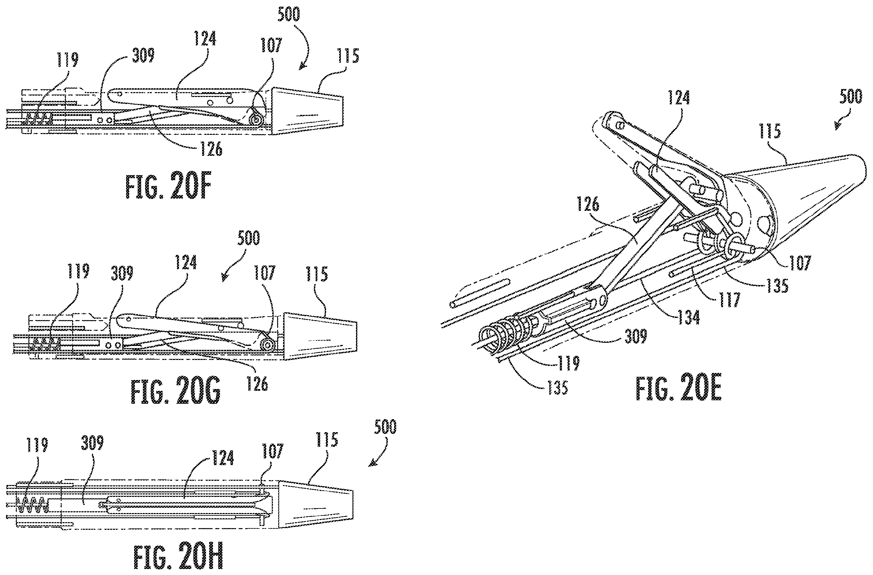

With reference to FIGs. 20A-20H, an exemplary pericardial tissue transection device (also referred to "medical device 500") for creating elongated incisions within a pericardium in accordance with the present claimed invention is provided. The elongated incisions may be completed with a "glide" action or a "scissor" action as depicted. Example medical devices, such as device 500, may use a "glide" action and/or a "scissor" action to cut along the wall of the pericardial cavity. The "glide" action maintains the first incision member 124 and the second incision in a fixed position (e.g., angle between the first incision member 124 and the second incision member 126 is fixed) while the incision assembly 101 is being moved along the pericardial. The "scissor" action is an instance in which the first incision member 124 and the second incision member 126 may move relative to one another during the cutting process (e.g., the angle between the first incision member 124 and the second incision member 126 is not fixed and may move to create a scissor force on the tissue).

Device 500 includes an incision assembly 101 having a longitudinal axis and at least one opening 136. The opening 136 may include an opening top defined at the top of the cavity in which the opening defines, and an opening bottom defined at the bottom of the cavity in which the opening defines. The incision assembly 101 is operably coupled to a distal end of an elongated body 129, such as a flexible and/or steerable catheter or catheter.

The incision assembly includes a first incision member 124 having a proximal end 124a and a distal end 124b, where the proximal end of the first incision member 124 is pivotably connected to the incision assembly. A second incision member 126 having a proximal end 126a and a distal end 126b, with the proximal end of the second incision member 126 pivotably connected to the first incision member 124 may also be provided, as shown. A first biasing member 117 operably coupled to the proximal end 124a of the first incision member 124 and a second biasing member 119 operatively coupled to the distal end 126b of the second incision member 126 provides for reciprocating and/or reversible operation of the two incision members 124, 126 (scissor blades) that can be user controlled.

The first incision member 124 and second incision member 126 may be moved between a retracted position (first configuration) and a deployed position (second configuration) as discussed herein. The retracted position is a position in which the first incision member 124 and the second incision member 126 are disposed within the opening 136. The deployed position is a position in which the first incision member 124 is at least partially protruding from the opening 136. The incision assembly 101 may have various locations in which the first incision member 124 and second incision member 126 can be in the deployed position. For example, the height of the protrusion of the first incision member 124 from the opening 136 may be adjusted and/or locked in place. The height may be based on the tissue to be cut.

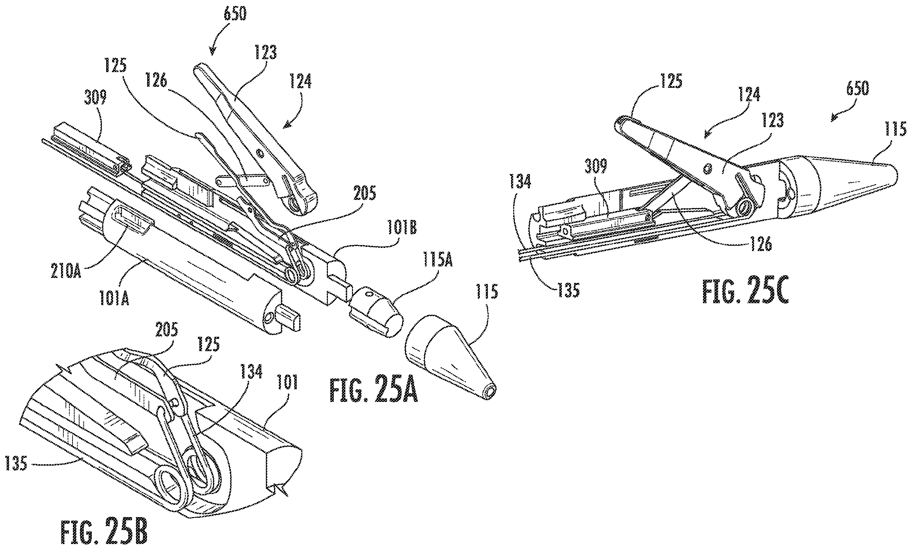

The two incision members 124, 126 may include sharpened blades, RF electrodes, or a combination. For example, the first incision member 124 may include a cutting tip 125 and one or more interior cutting surfaces (e.g., interior cutting surfaces 205A, 205B). Each of the cutting tip 125 and/or one or more interior cutting surfaces (e.g., interior cutting surfaces 205A, 205B in FIGs. 25A-25H and interior cutting surface 205 in FIGs. 25A-25C) may include an electrode, a blade, a sharpened edge, and/or the like configured to cut human tissue. In various embodiments, each of the cutting tip 125 and/or the interior cutting surfaces (e.g., interior cutting surfaces 205A, 205B in FIGs. 25A-25H and interior cutting surface 205 in FIGs. 25A-25C) may have a monopolar RF electrode or a bipolar RF electrode. In various embodiment, the cutting tip 125 may be a monopolar RF electrode (e.g., the cutting tip 125 may be an edge-only monopolar RF electrode that reduces danger to surrounding tissue during cutting process). In various embodiments, the interior cutting surfaces (e.g., interior cutting surfaces 205A, 205B in FIGs. 25A-25H and interior cutting surface 205 in FIGs. 25A-25C) may be bipolar RF electrodes (e.g., the interior cutting surfaces may have electrodes designed to cut the incision while being moved). The impedance may be measured between the interior cutting surfaces 205A, 205B, such that the impedance may be monitored during cutting to confirm the cut is occurring (e.g., the tissue being cut provide an impedance between the interior cutting surfaces 205A, 205B).

Additionally or alternatively, the first incision member 124 may have a blunt blade 201 in place of, or in addition to the interior cutting surface(s) 205A, 205B. As such, the blunt blade 201 may contact the wall of the pericardial wall and extend the incision via force of the movement of the incision assembly 101. The blunt blade 201 may provide real-time cut confirmation as the incision may be more easily seen by an operator.

The second incision member 126 may also include a cutting surface (e.g., facing in the direction of the interior cutting surfaces). The cutting surface may be a sharpened blade, RF electrode, or a combination thereof.

The incision assembly may also have one or more orientation markers 215 used to monitor the position of the incision assembly 101. The orientation markers 215 may be radiopaque markers that are visible via one or more imaging devices. In various embodiments, the orientation marker 215 may be configured into a pattern that indicates the direction of the incision assembly. As shown, the incision assembly has three orientation markers 215 in a triangle, such that the triangle points in the direction in which an incision may be made. As such, the operator may determine the direction in which the incision assembly 101 is located and into which surrounding tissue the first incision member 124 may be extended.

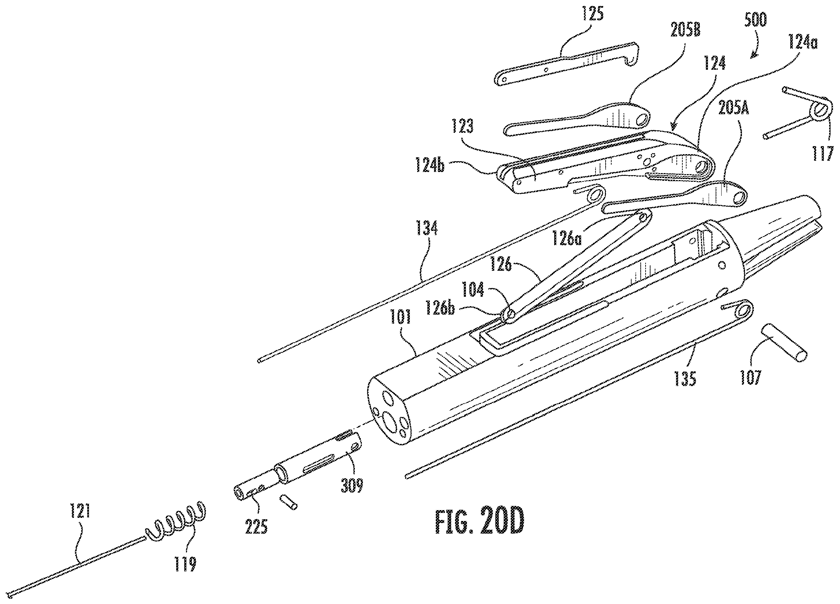

Device 500 is adaptably coupled to a controller 1000 described in further detail below. In one example, controller 1000 is operably coupled to the second incision member 126, for example, via an actuator rod 121 (shown in FIG. 20D) that functions to move the two incision members 124, 126. In various embodiments, as shown in FIG. 20D, the actuator rod 121 may be attached to a spring (second biasing member 119) to dampen the movement of the incision member 126. Additionally, a yoke 309 (with internal yoke biasing member 225) may be configured between the spring and the second incision member 126 for stability during movement of the actuator rod 121.

In one example, the first incision member 124 comprises a first cutting surface arranged in close proximity to a second cutting surface of the second incision member 126 so as to provide a scissoring action sufficient to cut pericardial tissue. As shown, the first incision member 124 and second incision member 126 are axially aligned along a longitudinal axis of the incision assembly 101. The distal end 124b of the first incision member 124 is reversibly adjustable laterally relative to the longitudinal axis of the incision assembly 101 between a range of angles so as to provide a scissoring action sufficient to cut pericardial tissue.

The proximal end 124a of the first incision member 124 is pivotably connected to the incision assembly 101 at a first connection point (shown by pin 107), and the proximal end 126a of the second incision member 126 is pivotably connected to the incision assembly 101 at a second connection point 104. The distal end of the second incision member 126 is pivotably connected to the first incision member 124 at third connection point 108. As shown, the third connection point 108 is positioned between the first connection point 106 and the distal end 124b of the first incision member 124, e.g., point 108 is spatially separated from pivot point 106.

A first biasing member 117 is operatively coupled to the incision assembly 101 and to the proximate end of the first incision member 124. The first biasing member 117 exerts a rotary force or torque to the first incision member 124, laterally projecting the distal end of the first incision member 124 through an opening 136 of the incision assembly 101. The second biasing member 119 exerts a pushing or spring force to the distal end 126b of the second incision member 126 via the yoke 309, pushing the distal end 126b of the second incision member 126 longitudinally towards the first biasing member 117.

In this configuration, distal end 126b of the second incision member 126 is reversibly adjustable parallel to the longitudinal axis of the incision assembly 101. With distal end 126b of the second incision member 126 operably coupled to actuator rod 121 (e.g., via the yoke 309, comes into contact with the second biasing member 119 connected to and moved by the actuator rod 121), translation of distal end 126b controlled by the controller via actuator rod 121, provides for reciprocation of the two incision members 124, 126 against one or both of the biasing forces of biasing members 117, 119. The first biasing member 117 can be at least one of a torsion spring and a compression spring. In various embodiments, the first biasing member 117 may be assisted or replaced by a biasing effect caused by one or more electrical wires 134, 135 (e.g., as shown the electrical wires 134, 135 may also provide a biasing force). The second biasing member 119 can be a spring.

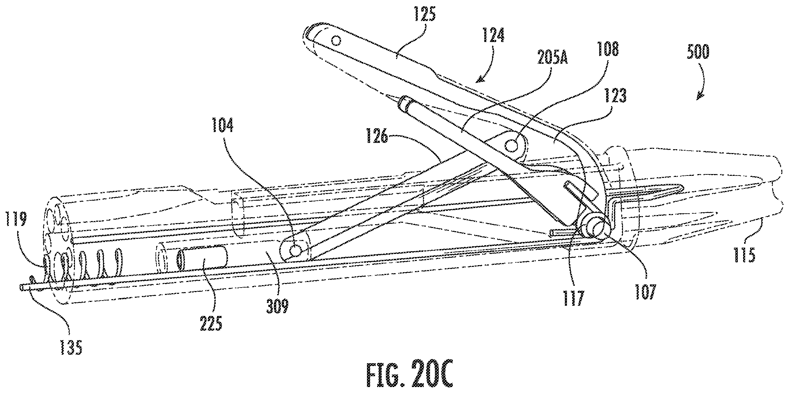

As shown in FIGs. 20C and 20D, distal end 126b of the second incision member 126 is operably connected to a yoke 309, where the yoke is configured to receive the distal end 126b of the second incision member 126. The yoke 309 may have an internal yoke biasing member 225 that positioned within the yoke 309 to move along the yoke 309 during movement of the yoke 309 (e.g., in response to contact with the second biasing member 119).

Medical device 500 can further comprise a sheath (not shown), where the sheath is slidably traversable over an outer diameter of the incision assembly 101. Thus, with a sheath, a first configuration, where the sheath is located over the at least one opening 136 of the incision assembly 101 is provided, where in the first configuration the sheath counteracts the bias of the first biasing member 117 and/or the electrical wires 134, 135 and prevents lateral projection of the first incision member 124 from the incision assembly 101. The medical device of any one of the embodiments herein may also have a second configuration. In such a second configuration, the at least one opening of the incision assembly 101 is uncovered, laterally projecting the first incision member 124 through the at least one opening 136 of the incision assembly 101.

In one example, the transection device is operated by manipulating the controller so as to exert a pulling force to the actuator rod 121, counteracting the force of the second biasing member 119 and traversing the distal end 126b of the second incision member 126 parallel to the longitudinal axis of the incision assembly 101. During this manipulation, the proximate end 126a of the second incision member 126 counteracts the force of the first biasing member 117 and/or electrical wires 134, 135, reducing the lateral projection of the first incision member 124 resulting in a scissoring action by pulling and then releasing the actuator rod 121.

In one example, the distal end 124b of the first incision member 124 comprises a cutting tip 125. In various embodiments, the cutting tip 125 may be an electrically conductive tip operably coupled to an independent source of radiofrequency energy or electrical current. In this manner, the tip 125 can be operated independently of first incision member 124, as the first incision member 124 includes a non-conductive element 123 electrically isolating tip 125. In this configuration, the opposite sides of the first incision member 124 are independently operably coupled to independent sources of radiofrequency energy or electrical current via wires 134, 135. In one example, the controller is configured to independently operate the source of radiofrequency energy or electrical current to the first incision member 124 and the second incision member 126 and the conductive tip 125.

The transection device 500 may have one or more stabilizing members located adjacent to the at least one opening of the incision assembly 101. In one example, the one or more stabilizing members reversibly projects laterally about 120 degrees radially apart about the incision assembly. The one or more stabilizing members can be a wire, loop, or shape-memory metal, or one or more inflatable structures. In one example, the one or more stabilizing members prevents rotation of the incision assembly 101 around the longitudinal axis.

In various embodiments, the medical device 500 may have one or more suction devices. For example and in accordance with the present claimed invention, the suction lumens 210A, 210B may be disposed on the incision assembly 101). In various embodiments, the incision assembly 101 includes one or more suction lumens 210A, 210B. The suction lumen(s) 210A, 210B may be configured to provide a suction force that is capable of engaging with the wall of the pericardial cavity 54. In various embodiments, the suction lumen(s) 210A, 210B may provide sufficient force to maintain close contact between the incision assembly 101 and the wall of the pericardial cavity, while still allowing the incision assembly 101 to be moved along the pericardial cavity (e.g., to allow the incision to be made). As such, the suction lumen(s) 210A, 210B may provide a constant and/or near constant suction force in the direction of the wall of the pericardial cavity and need not necessarily maintain contact between the suction lumen(s) 210A, 210B and the wall of the pericardial cavity. In various embodiments, the suction force via the suction lumen(s) 210A, 210B may be applied during the initial incision creation (e.g., in an instance in which the first incision member begins to protrude from the opening 136 and the cutting tip 125 begins to make the incision to the wall of the pericardial cavity). Upon creation of the incision, the suction force may be maintained (e.g., kept constant during the movement of the incision assembly), reduced, or terminated (e.g., the incision assembly 101 may be able to maintain positioning along the incision with the scissor effect of the first incision member 124 and the second incision member 126).

In various embodiments, the suction lumen(s) 210A, 210B may be parallel to or otherwise adjacent to the opening 136 of the incision assembly 101. While the example embodiments illustrate two suction lumens 210A, 210B, various embodiments may have more or less suction lumens (e.g., more suction lumens may be provided to provide a higher suction force and stability).

In various embodiments, the length of the openings for the suction lumens 210A, 210B may be less than the longitudinal length of the opening 136. Alternatively, the openings for the suction lumens 210A, 210B may be at least or greater than the longitudinal length of the opening 136. In various embodiments, a given suction lumen may have a plurality of openings provided in the incision assembly (e.g., parallel to the opening 136). In various embodiments, the first suction lumen 210A and the second suction lumen 210B may be positioned at opposite sides of the opening 136. In various embodiments, the first suction lumen 210A and the second suction lumen 210B may be positioned parallel to one another along the opening 136. For example, the first suction lumen 210A and the second suction lumen 210B may be positioned at the same position along the longitudinal direction of the incision assembly 101 at opposite sides of the opening 136.

The one or more suction lumens 210A, 210B may include one or more suction cups provided in the suction lumens configured to engage with the wall of the pericardial cavity 54. The one or more suction lumens 210A, 210B may be contained within and/or protrude from the opening of the given suction lumen. In various embodiments, the suction cup(s) of the suction lumens 210A, 210B may be folded within the given suction lumen 210A, 210B for transport. For example, the suction cup(s) may be folded in an instance in which the sheath is covering the opening of the suction lumens 210A, 210B. The suction cups discussed herein may be any material provided in addition to the incision assembly 101 that assists the suction between the incision assembly 101 and the wall of the pericardial cavity.

The one or more suction lumen(s) 210A, 210B may be connected to a vacuum source via the lumens provided in catheter 129. The vacuum source may provide the one or more suction lumens with a suction force that engages the wall of the pericardial cavity. The suction force may be sufficient to maintain the position of the incision assembly 101 during the cutting process. Various stabilizing member(s) discussed herein may also assist the one or more suction lumen(s) 210A, 210B to maintain connection with the pericardial cavity (e.g., the stabilizing member may engage the opposite wall of the pericardial cavity). The suction force may be actuated and/or otherwise controlled by a controller, such as the controllers discussed herein.

In various embodiments, the suction lumen(s) 210A, 210B may be provided as a cavity within the incision assembly 101 (e.g., a cavity connected to a vacuum source as discussed above). Additionally, the suction lumen(s) 210A, 210B may include one or more materials to assist with suction between the suction lumen and the pericardial wall. For example, one or more of the suction lumen(s) 210A, 210B may have a suction cup configured to engage with the wall of the pericardial cavity 54. Various other materials may be provided to assist the suction between the suction lumen(s) 210A, 210B and the wall of the pericardial cavity 54.

Materials suitable for the suction cups provided in an example suction lumen 210A, 210B may include, without limitation, thermoplastic elastomers, EPDM rubbers, thermoset rubbers, polysilicones, polysiloxanes, polyurethanes, polyvinyl chlorides, styrene-ethylenebutylene-styrenes, and polytetrafluoroethylenes, derivatives, copolymers, and blends thereof. For example, the suction lumen(s) 210A, 210B may be a suction cup(s) made out of silicone.

Upon positioning of the incision assembly 101 within the pericardial cavity 54, the incision assembly may be moved from the retracted position to the deployed position as discussed herein. The first incision member 124 (e.g., via the tip 125), using a blade, an electrode, and/or the like, punctures into the pericardial tissue allowing the incision to be made along the pericardium tissue (e.g., the parietal layer 55). The incision is made by the first incision member 124 and/or the second incision member 126, using a blade, an electrode, and/or combination thereof. The incision length may be based on the length of the cutting surface 310. As shown, the catheter 129 maintains the same position within the pericardial cavity 54 due to the suction device(s) and/or the stabilizing member(s). As such, the incision may be a linear incision that is the length of the cutting surface 310 that is engaging the parietal layer 55. The multi-lumen catheter 129 remains stationary within the pericardial cavity 54 during the incision by the cutting surface.

Upon completing the intended incision, the cutting surface 310 is moved back to the retracted position. The stabilizing member(s) may be moved to the stabilizing retracted position. The suction force provided to the suction lumen(s) 210A, 210B may be reduced to cause the suction between the suction device(s) and the parietal layer to dissipate. The sheath can be moved to cover the incision opening 136. The multi-lumen catheter can then be removed from the human body without having any blades exposed.

Referring now to FIG. 21, various lumens in an incision assembly 101 to be connected to a catheter in accordance with various embodiments is provided. As such, the catheter is operably coupled to the incision assembly 101. As such, the catheter 129 may have the same lumen configuration as the incision assembly 101. The catheter 129 may be a multi-lumen catheter. The catheter may have one or more lumens for various operations discussed herein. For example, the lumens provided in a catheter and continued in the incision assembly 101 may include suction lumen(s) 210A, 210B; camera lumen(s) 302 (e.g., to receive a camera); electrode lumen(s) (e.g., lumens to receive the electrical wires 134, 135); actuating rod lumen(s) (e.g., lumen to receive the actuating rod (not shown) and the second biasing member 119); and/or other various lumens to allow for the incision assembly 101, catheter, and/or the like to operator. For example, an optical channel or lumen (e.g., camera lumen 302) may be provided in the catheter to accommodate a lens coupled to a fiber optic cable, optionally with a light source, e.g., an LED. In one example, the presently disclosed method further comprises obtaining visual information during accessing, traversal of the pericardial cavity, exiting and/or cutting, for example, using an optical channel in the catheter to accommodate a lens coupled to a fiber optic cable, optionally with a light source, e.g., an LED. The camera and/or light source may be sized to fit within the medical device. For example, the camera and/or light source may be 1 millimeter or less in diameter.

Various other lumens may be provided and/or used for the various operations discussed herein. Additionally, the catheter may have one or more catheters not used by the operations herein (e.g., the catheter may be a universal multi-lumen catheter with additional lumens not necessary for the operations herein.

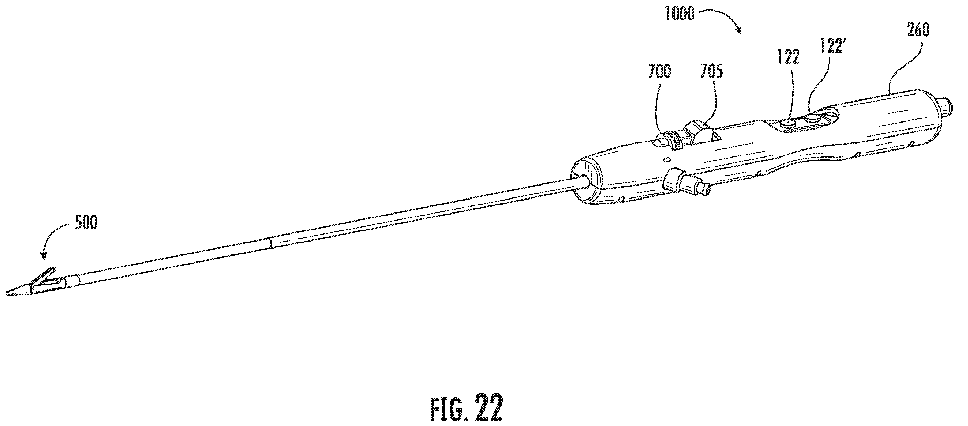

With reference to FIG 22, an example controller 1000 is shown having handle 260, actuating buttons 122, 122' for operably coupling with the medical devices of various embodiments (e.g., medical device 500, medical device 600, medical device 650, etc.). For example, the controller 1000 may be used to actuate and/or otherwise controller the actuator rod 121, retracting sheath 130, suction lumens, guidewire, extending stabilizing members 120 via rods 121, etc. In various embodiments, the controller 1000 facilitates various potential operations of the incision assembly 101 components, including biasing/clamping the distal blade open and closed, which can be achieved by an appropriate mechanism configured to pull/push a rod. In various embodiments, there is a mechanism used to provide a suction force to the one or more suction lumens 210A, 210B. In various embodiments, there is a mechanism used to release/retrieve balloons/nitinol components that function to stabilize and apply counterpressure for the incision assembly 101 and its components, including the cutting surface. Controller 1000 may include one or more buttons 122, 122' used to operate and control the electrosurgical features of the device, e.g., current and/or RF.

The controller 1000 may also have one or more actuating knobs 700, 705 for controlling the catheter 129 and the various components of the transection device. Knob 700 may be configured to rotate the flexible catheter 129 in response to orientation information derived from fluoroscopy or other visualization means. Knob 705 may be engaged to activate one or more components on the medical device (e.g., providing a suction force to the suction lumens, actuating the stabilizing member(s), etc.). Various other controllers are envisioned that allow for the aforementioned transection devices to be deployed and manipulated.

A method of manipulating medical device 100 would include providing medical device 100 engaged with a controller and controlling, for example, manipulating any one or combination of actuating knobs and/or actuating buttons to provide at least one of suction to the one or more suction lumens, movement of the first incision member and the second incision member between the retracted position and the deployed position, movement of the incision assembly, energy to the incision device, or movement of one or more stabilizing members with the controller 1000. The aforementioned method is applicable to any previously disclosed transection devices.

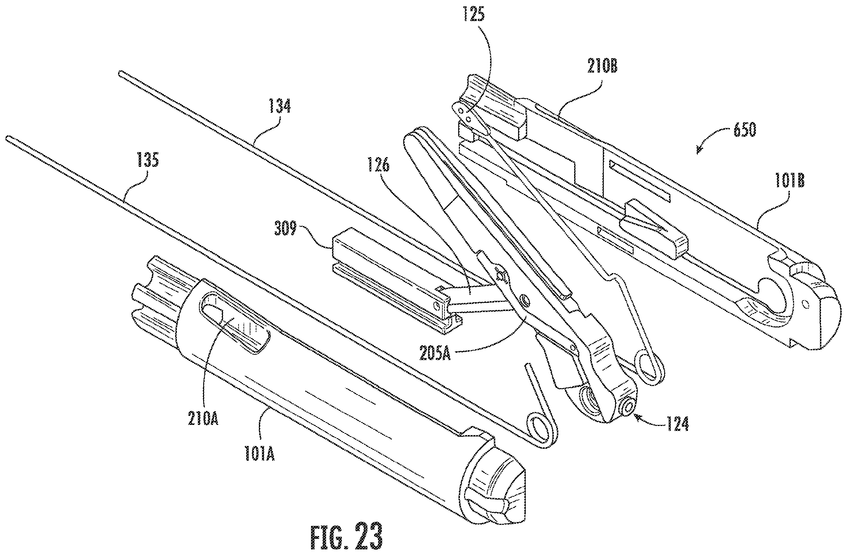

Referring now to FIG. 23, an exploded view of an incision device in accordance with various embodiments is provided. Unless otherwise noted, the medical device 650 shown in FIGs. 23, 24A-24C, 25A-25C, 26A-26B, 27, and 28A-28C include the various components and/or functionality of the medical device 500 discussed in reference to FIGS. 20A-20H.

As, shown, the incision assembly 101 may include a first incision member 124 and a second incision member 126 operably coupled to one another such that the movement of the second incision member 126 (via the yoke 309 and the actuating rod (not pictured)) causes the first incision member 124 to also move (e.g., causing a scissor effect as discussed herein). Additionally, the incisions assembly 101 may include electrical wires 134, 135 that may provide an electrical current to one or more electrodes (e.g., interior cutting surface 205 and tip 125). The electrical wire(s) 134, 135 may also provide a biasing force against the first incision member 124 (e.g., pushing the first incision member 124 out of the opening 136).

In various embodiments, the incision assembly may have two separate incision assembly components 101A, 101B that are configured to engage one another. As shown in FIG. 23, the yoke 309 may define one or more channels configured to engage with one or more grooves of the incision assembly components 101A, 101B. As such, the yoke 309 may move along the incision channel (within the opening 136) of the incision assembly. As such, the second incision member 126 is moved based on the movement of the yoke 309 as discussed herein.

The suction lumen(s) 210A, 210B may be provided in the incision assembly components 101A, 101B. For example, the first suction lumen 210A is provided in the first incision assembly component 101A and the second suction lumen 210B is provided in the second incision assembly component 101B.

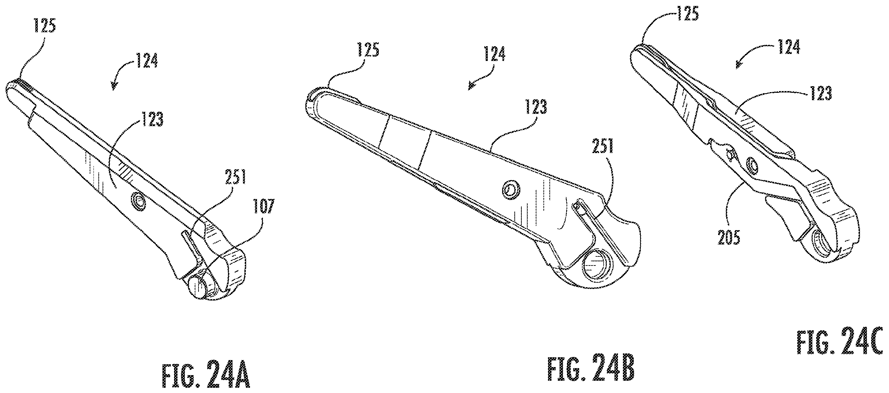

Referring now to FIGs. 24A, 24B, and 24C, various views of a first incision member 124 of various embodiments, such as the medical device 650 shown in FIG. 23 is provided. The first incision member 124 shown in FIGs. 24A, 24B, and 24C may be used in various different embodiments discussed herein unless otherwise stated. As shown, the first incision member may include a non-conductive element 123 configured to receive the cutting tip 125 and one or more interior cutting surfaces 205 (e.g., an electrode). The non-conductive element 123 may be an insulating material (e.g., ceramic). The non-conductive element may be ceramic due to the dielectric strength and minimal heat conductivity of ceramic.

The interior cutting surface 205 is configured to be received by a cavity within the non-conductive element 123. The non-conductive element 123 may also have one or more cavities (e.g., wire cavity 251) to receive the electrical wires 134, 135. For example, the end of the electrical wire 134 is connected to the interior cutting surface 205 and additionally provides a biasing force against the wire cavity 251. The non-conductive element 123 may also define a cavity to receive the cutting tip 125 (e.g., the cutting tip 125 may be at least partially disposed within the non-conductive element 123 with the edge of the tip 125 protruding at the distal end of the first incision member 124).

Referring now to FIGs. 25A-25C, FIG. 25A shows an exploded view of another embodiment of the example medical device 650 (e.g., an incision assembly). FIG. 25B illustrates a close-up view of the connection between the electrical wires 134, 135 and the interior cutting surface 205 and the cutting tip 125. FIG. 25C illustrates the incision assembly in the deployed position. The medical device 650 may have the same or similar internal configuration as the medical device 600 discussed above in reference to FIG. 23.

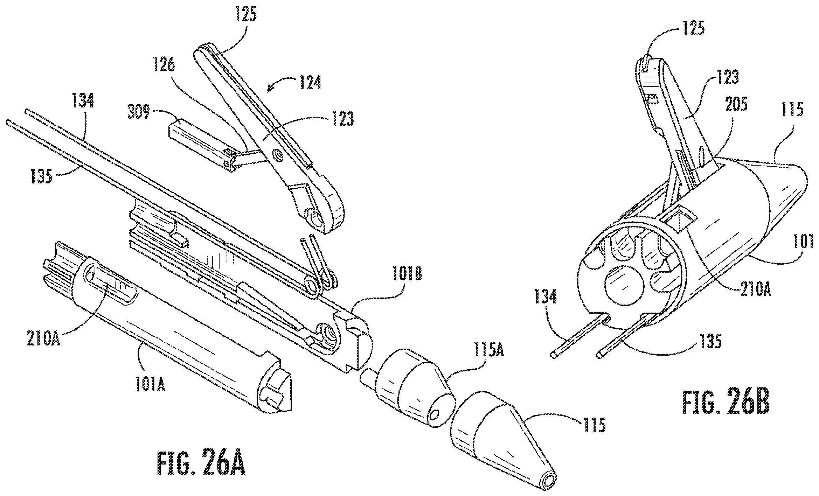

In various embodiments, the incision assembly components 101A, 101B may define different protrusions and/or receivers for the introducer 115 to be attached thereon. For example, the incision assembly components 101A, 101B may have different shaped protrusions (e.g., FIG. 25A has a different protrusion shape than FIG. 26A). In various embodiments, the introducer 115 may have an interior introducer 115A that is attached to the introducer 115. Additionally, the introducer (and the interior introducer 115A) may have an aperture configured to allow a guidewire to pass through, as discussed in various embodiments herein.

Referring to FIG. 25B, a zoomed-in view of the electrical connections for each of one or more electrode (e.g., interior cutting surface 205 and cutting tip 125) of the medical device shown in FIG. 25A is provided. As discussed herein, the electrical wires may provide a current to the given electrodes (e.g., interior cutting surface 205 and/or cutting tip 125). The electrical wires 134, 135 may also include a biasing member (e.g., such as the spring shown in FIG. 25B) to provide an opening force to the first incision member 124.

Referring to FIG. 26A and 26B, an example medical device in accordance with various embodiments. FIG. 26A illustrates an exploded view of the example medical device. As shown, the medical device of FIG. 26A may have the components discussed herein in reference to any of the embodiments (e.g., FIG. 25A). Additionally, the introducer 115 may be different in various embodiments. For example, the introducer 115 shown in FIG. 26A has an interior introducer 115A. Each of the introducer and the interior introducer 115A may include an aperture (e.g., a continuation of the guidewire lumen discussed herein) in which a guidewire (e.g., guidewire 113 shown in various embodiments) may pass through.

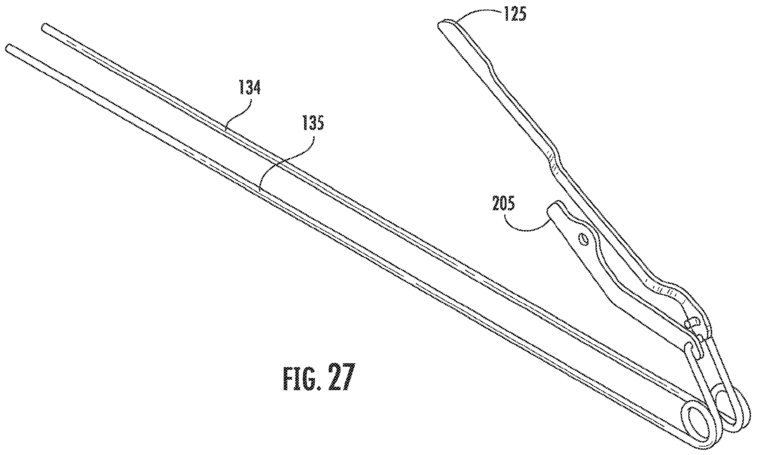

Referring now to FIG. 27, the connection between electrical wires 134, 135 and the interior cutting surface 205 and cutting tip 125 in accordance with various embodiments is shown. The configuration may be used in various embodiments. As shown, the cutting tip 125 may be an electrode. Additionally or alternatively, the cutting tip 125 may include a sharp edge or blade structure. The cutting tip 125 upon deployment is configured to make contact with the wall of the pericardial cavity 54, creating the incision in the wall of the pericardial cavity. Upon making the initial incision, the incision may be continued until at least a portion of the interior cutting surface 205 is exposed to the wall of the pericardial cavity. In such an instance, the electrical current provided to the cutting tip 125 may be discontinued (or continued for a short time).

The interior cutting surface 205, which may include a blade, sharp edge, electrode, and/or other cutting surfaces may be used to continue the incision along the wall of the pericardial cavity. A cutting surface on the second incision member 126 may also be used to continue the incision (e.g., the scissor cutting effect discussed herein). The electrical current provided to the cutting tip 125 and the interior cutting surface 205 may be independently controlled (e.g., the current may be provided to the cutting tip 125 for the initial incision creation, but only to the interior cutting surface 205 during the movement of the incision assembly 101 along the incision).

As discussed above, in an example embodiment, the cutting tip 125 may be a monopolar RF electrode and the interior cutting surface 205 may be a bipolar RF electrode.

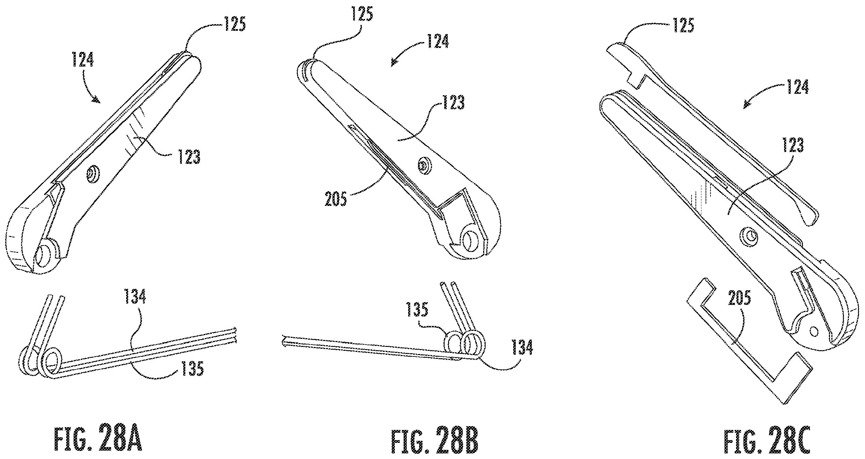

Additionally, FIGs. 28A, 28B, and 28C illustrate the connection between the electrical wires 134, 135 and the cutting tip 125 and interior cutting surface 205 along with the configuration of the cutting tip 125, the non-conductive element 123, and the interior cutting surface 205 in the first incision member 124. The first incision member 124 shown in FIGs. 28-28C may be used in various embodiments discussed herein. Additionally, the configuration of the first incision member 124 of FIGS. 28A-28C may be substantially the same as the first incision member 124 of FIGs. 24A-24C.

In various embodiments, one or more of the electrical wires 134, 135 may include a biasing member, such as a spring. As shown, the electrical wires 134, 135 may include a coiled wire that also acts as a spring. In such an embodiment, the electrical wires 134, 135 may provide a force against the first incision member 124 in the direction opposite of the opening 136 of various embodiment (e.g., providing an opening force causing the first incision member 124 to move to the deployed position).

While certain embodiments of the present disclosure have been illustrated with reference to specific combinations of elements, various other combinations may also be provided without departing from the teachings of the present disclosure. Thus, the present disclosure should not be construed as being limited to the particular exemplary embodiments described herein and illustrated in the Figures but may also encompass combinations of elements of the various illustrated embodiments and aspects thereof.

The invention is defined by the following claims.

## Claims

1. A medical device (500), the device comprising:
an incision assembly (101) configured to be operably coupled to a distal end of a catheter (129), wherein the incision assembly comprises an incision opening (136) defined along the incision assembly and one or more suction lumens (210A, 210B) defined along the incision assembly, wherein the suction lumens provide a suction force in a direction in which the first incision member extends out of the incision opening; and
a retractable cutting apparatus disposed within the incision opening, wherein the retractable cutting apparatus comprises:
a first incision member (124) comprising at least one cutting surface (105), wherein the at least one cutting surface faces away from the distal end of the catheter and towards a proximal end of the catheter, and wherein the first incision member defines a first member distal end (124b) and a first member proximal end (124a), wherein the first member proximal end is rotatably fixed proximate to a distal end of the incision opening;
a second incision member (126) defining a second member distal end (126b) and a second member proximal end (126a), wherein the second member proximal end is attached to the first incision member at a first member proximal end;
wherein the retractable cutting apparatus is configured to move between a retracted position and a deployed position, wherein in an instance in which the retractable cutting apparatus is in the retracted position, the first incision member is within the incision opening,
wherein in an instance in which the retractable cutting apparatus is in the deployed position, as least a portion of the first incision member protrudes from the incision opening.

2. The medical device of Claim 1, wherein the at least one cutting surface of the first incision member comprises at least one interior cutting surface or a cutting tip.

3. The medical device of any one of Claim 1 or Claim 2, wherein at least one of the at least one cutting surface of the first incision member:
a) is an electrode, optionally wherein one or more of the at least one cutting surface of the first incision member comprises a monopolar electrode or a bipolar electrode; and/or
b) comprises a blunt blade.

4. The medical device of any one of Claims 1-3, wherein the second incision member comprises at least one cutting surface, wherein the at least one cutting surface of the second incision member is facing the opposite direction of at least one of the at least one cutting surface of the first incision member.

5. The medical device of any one of Claim 1-4, wherein the one or more suction lumens comprise a first suction lumen defined along a longitudinal axis of the incision assembly and a second suction lumen parallel to the first suction lumen in the longitudinal axis of the incision assembly.

6. The medical device of any one of Claim 1-5, further comprising one or more orientation markers (215) provided on the incision assembly, wherein the one or more orientation markers indicates a cutting direction of the incision assembly, and wherein the one or more orientation markers are radiopaque.

7. The medical device of any one of Claim 1-6, further comprising a camera provided via a camera lumen (302) in the catheter, wherein the camera is capable of providing one or more images of the retractable cutting apparatus.

8. The medical device of any one of Claim 1-7, further comprising one or more electrical wires (134) configured to provide an electrical current to one or more of the at least one cutting surface of the first incision member, wherein the one or more electrical wires provide a biasing force to the first incision member,
optionally wherein the at least one of the one or more electrical wires comprise a torsion spring configured to provide the biasing force to the first incision member.

9. The medical device of any one of Claims 1-8, further comprising an actuator rod (121) in communication with the second member distal end of the second incision member, wherein the actuator rod is configured to move the first incision member and the second incision member.

10. The medical device of Claim 9, further comprising a yoke (309) attached to the second member distal end of the second incision member, wherein the yoke is configured to receive the force from the actuator rod or a biasing force attached to the actuator rod.

11. The medical device of any one of Claims 1-10, further comprising a sheath (130) configured to movably cover the incision opening.

12. The medical device of Claim 11, wherein the sheath is configured to provide a counterforce to the biasing force caused by the one or more electrical wires in an instance in which the sheath is at least partially covering the incision opening.

13. The medical device of any one of Claims 1-12, wherein the at least one cutting surface of the first incision member comprises a first interior cutting surface (205A) and a second interior cutting surface (205B), wherein the first interior cutting surface and the second interior cutting surface are both electrodes, and wherein an impedance between the first interior cutting surface and the second interior cutting surface indicates an instance in which a material is being cut by the first interior cutting surface and the second interior cutting surface.

14. The medical device of any one of Claims 1-13, further comprising a controller (1000) engaged to the incision assembly or retractable cutting apparatus.

15. The medical device of Claim 14, wherein the controller is configured to provide at least one of suction to the one or more suction lumens, movement of the first incision member and the second incision member between the retracted position and the deployed position, movement of the incision assembly, energy to the incision assembly, or movement of one or more stabilizing members.

## Patentansprüche

1. Medizinische Vorrichtung (500), wobei die Vorrichtung Folgendes umfasst:
eine Inzisionsanordnung (101), die dazu ausgelegt ist, mit einem distalen Ende eines Katheters (129) wirkgekoppelt zu werden, wobei die Inzisionsanordnung eine entlang der Inzisionsanordnung definierte Inzisionsöffnung (136) und ein oder mehrere entlang der Inzisionsanordnung definierte Sauglumen (210A, 210B) umfasst, wobei die Sauglumen eine Saugkraft in einer Richtung bereitstellen, in der sich das erste Inzisionselement aus der Inzisionsöffnung erstreckt; und eine einfahrbare Schneidvorrichtung, die innerhalb der Inzisionsöffnung angeordnet ist, wobei die einfahrbare Schneidvorrichtung Folgendes umfasst:
ein erstes Inzisionselement (124), das mindestens eine Schneidfläche (105) umfasst, wobei die mindestens eine Schneidfläche vom distalen Ende des Katheters weg und zu einem proximalen Ende des Katheters weist, und wobei das erste Inzisionselement ein distales Ende (124b) des ersten Elements und ein proximales Ende (124a) des ersten Elements definiert, wobei das proximale Ende des ersten Elements in der Nähe eines distalen Endes der Inzisionsöffnung drehbar befestigt ist;
ein zweites Inzisionselement (126), das ein distales Ende (126b) des zweiten Elements und ein proximales Ende (126a) des zweiten Elements definiert, wobei das proximale Ende des zweiten Elements an einem proximalen Ende des ersten Elements an dem ersten Inzisionselement angebracht ist;
wobei die einfahrbare Schneidvorrichtung dazu ausgelegt ist, sich zwischen einer eingefahrenen Position und einer ausgefahrenen Position zu bewegen, wobei sich in einem Fall, in dem sich die einfahrbare Schneidvorrichtung in der eingefahrenen Position befindet, das erste Inzisionselement innerhalb der Inzisionsöffnung befindet,
wobei in einem Fall, in dem sich die einfahrbare Schneidvorrichtung in der ausgefahrenen Position befindet, mindestens ein Teil des ersten Inzisionselements aus der Inzisionsöffnung vorsteht.

2. Medizinische Vorrichtung nach Anspruch 1, wobei die mindestens eine Schneidfläche des ersten Inzisionselements mindestens eine innere Schneidfläche oder eine Schneidspitze umfasst.

3. Medizinische Vorrichtung nach einem der Ansprüche 1 oder 2, wobei mindestens eine der mindestens einen Schneidfläche des ersten Inzisionselements:
a) eine Elektrode ist, wobei optional eine oder mehrere der mindestens einen Schneidfläche des ersten Inzisionselements eine unipolare Elektrode oder eine bipolare Elektrode umfassen; und/oder
b) eine stumpfe Klinge aufweist.

4. Medizinische Vorrichtung nach einem der Ansprüche 1-3, wobei das zweite Inzisionselement mindestens eine Schneidfläche umfasst, wobei die mindestens eine Schneidfläche des zweiten Inzisionselements der entgegengesetzten Richtung von mindestens einer der mindestens einen Schneidfläche des ersten Inzisionselements zugewandt ist.

5. Medizinische Vorrichtung nach einem der Ansprüche 1-4, wobei das eine oder die mehreren Sauglumen ein erstes Sauglumen, das entlang einer Längsachse der Inzisionsanordnung definiert ist, und ein zweites Sauglumen, das parallel zu dem ersten Sauglumen in der Längsachse der Inzisionsanordnung ist, umfassen.

6. Medizinische Vorrichtung nach einem der Ansprüche 1-5, ferner umfassend einen oder mehrere Orientierungsmarker (215), die an der Inzisionsanordnung bereitgestellt sind, wobei der eine oder die mehreren Orientierungsmarker eine Schneidrichtung der Inzisionsanordnung angeben und wobei der eine oder die mehreren Orientierungsmarker strahlenundurchlässig sind.

7. Medizinische Vorrichtung nach einem der Ansprüche 1-6, ferner umfassend eine Kamera, die über ein Kameralumen (302) in dem Katheter bereitgestellt ist, wobei die Kamera in der Lage ist, ein oder mehrere Bilder der einfahrbaren Schneidevorrichtung bereitzustellen.

8. Medizinische Vorrichtung nach einem der Ansprüche 1-7, ferner umfassend einen oder mehrere elektrische Drähte (134), die dazu ausgelegt sind, für eine oder mehrere der mindestens einen Schneidfläche des ersten Inzisionselements einen elektrischen Strom bereitzustellen, wobei der eine oder die mehreren elektrischen Drähte eine Vorspannkraft auf das erste Inzisionselement bereitstellen,
wobei optional der mindestens eine des einen oder der mehreren elektrischen Drähte eine Torsionsfeder umfasst, die dazu ausgelegt ist, die Vorspannkraft auf das erste Inzisionselement bereitzustellen.

9. Medizinische Vorrichtung nach einem der Ansprüche 1-8, ferner umfassend eine Betätigungsstange (121) in Verbindung mit dem distalen Ende des zweiten Elements des zweiten Inzisionselements, wobei die Betätigungsstange dazu ausgelegt ist, das erste Inzisionselement und das zweite Inzisionselement zu bewegen.

10. Medizinische Vorrichtung nach Anspruch 9, die ferner ein Joch (309) umfasst, das an dem distalen Ende des zweiten Elements des zweiten Inzisionselements angebracht ist, wobei das Joch dazu ausgelegt ist, die Kraft von der Betätigungsstange oder eine Vorspannkraft, die an der Betätigungsstange angebracht ist, aufzunehmen.

11. Medizinische Vorrichtung nach einem der Ansprüche 1-10, ferner umfassend eine Hülle (130), die dazu ausgelegt ist, die Inzisionsöffnung beweglich zu bedecken.

12. Medizinische Vorrichtung nach Anspruch 11, wobei die Hülle dazu ausgelegt ist, eine Gegenkraft zu der Vorspannkraft bereitzustellen, die durch den einen oder die mehreren elektrischen Drähte in einem Fall verursacht wird, in dem die Hülle die Inzisionsöffnung mindestens teilweise bedeckt.

13. Medizinische Vorrichtung nach einem der Ansprüche 1-12, wobei die mindestens eine Schneidfläche des ersten Inzisionselements eine erste innere Schneidfläche (205A) und eine zweite innere Schneidfläche (205B) umfasst, wobei die erste innere Schneidfläche und die zweite innere Schneidfläche beide Elektroden sind, und wobei eine Impedanz zwischen der ersten inneren Schneidfläche und der zweiten inneren Schneidfläche einen Fall angibt, in dem ein Material durch die erste innere Schneidfläche und die zweite innere Schneidfläche geschnitten wird.

14. Medizinische Vorrichtung nach einem der Ansprüche 1-13, die ferner eine Steuereinheit (1000) umfasst, die mit der Inzisionsanordnung oder der einfahrbaren Schneidvorrichtung in Eingriff steht.

15. Medizinische Vorrichtung nach Anspruch 14, wobei die Steuereinheit dazu ausgelegt ist, mindestens eines von Saugen für das eine oder die mehreren Sauglumen, Bewegung des ersten Inzisionselements und des zweiten Inzisionselements zwischen der eingefahrenen Position und der ausgefahrenen Position, Bewegung der Inzisionsanordnung, Energie für die Inzisionsanordnung oder Bewegung eines oder mehrerer Stabilisierungselemente bereitzustellen.

## Revendications

1. Dispositif médical (500), le dispositif comprenant :
un ensemble d'incision (101) configuré pour être couplé de manière fonctionnelle à une extrémité distale d'un cathéter (129), l'ensemble d'incision comprenant une ouverture d'incision (136) définie le long de l'ensemble d'incision et une ou plusieurs lumières d'aspiration (210A, 210B) définies le long de l'ensemble d'incision, les lumières d'aspiration fournissant une force d'aspiration dans une direction selon laquelle le premier élément d'incision s'étend hors de l'ouverture d'incision ; et
un appareil de coupe rétractable disposé à l'intérieur de l'ouverture d'incision, l'appareil de coupe rétractable comprenant :
un premier élément d'incision (124) comprenant au moins une surface de coupe (105), l'au moins une surface de coupe étant orientée à l'opposé de l'extrémité distale du cathéter et vers une extrémité proximale du cathéter, et le premier élément d'incision définissant une extrémité distale de premier élément (124b) et une extrémité proximale de premier élément (124a), l'extrémité proximale de premier élément étant fixée de manière rotative à proximité d'une extrémité distale de l'ouverture d'incision ;
un deuxième élément d'incision (126) définissant une extrémité distale de deuxième élément (126b) et une extrémité proximale de deuxième élément (126a), l'extrémité proximale de deuxième élément étant fixée au premier élément d'incision au niveau d'une extrémité proximale de premier élément ;
dans lequel l'appareil de coupe rétractable est configuré pour se déplacer entre une position rétractée et une position déployée ; le premier élément d'incision se trouvant à l'intérieur de l'ouverture d'incision dans le cas où l'appareil de coupe rétractable se trouve dans la position rétractée,
dans lequel au moins une partie du premier élément d'incision fait saillie hors de l'ouverture d'incision dans le cas où l'appareil de coupe rétractable se trouve dans la position déployée.

2. Dispositif médical selon la revendication 1, dans lequel l'au moins une surface de coupe du premier élément d'incision comprend au moins une surface de coupe intérieure ou une pointe de coupe.

3. Dispositif médical selon l'une quelconque des revendications 1 ou 2, dans lequel au moins l'une de l'au moins une surface de coupe du premier élément d'incision :
a) est une électrode, facultativement une ou plusieurs de l'au moins une surface de coupe du premier élément d'incision comprenant une électrode monopolaire ou une électrode bipolaire ; et/ou
b) comprend une lame émoussée.

4. Dispositif médical selon l'une quelconque des revendications 1 à 3, dans lequel le deuxième élément d'incision comprend au moins une surface de coupe, l'au moins une surface de coupe du deuxième élément d'incision étant orientée dans la direction opposée à celle d'au moins l'une de l'au moins une surface de coupe du premier élément d'incision.

5. Dispositif médical selon l'une quelconque des revendications 1 à 4, dans lequel la ou les lumières d'aspiration comprennent une première lumière d'aspiration définie le long d'un axe longitudinal de l'ensemble d'incision et une deuxième lumière d'aspiration parallèle à la première lumière d'aspiration dans l'axe longitudinal de l'ensemble d'incision.

6. Dispositif médical selon l'une quelconque des revendications 1 à 5, comprenant en outre un ou plusieurs marqueurs d'orientation (215) disposés sur l'ensemble d'incision, le ou les marqueurs d'orientation indiquant une direction de coupe de l'ensemble d'incision, et le ou les marqueurs d'orientation étant radio-opaques.

7. Dispositif médical selon l'une quelconque des revendications 1 à 6, comprenant en outre une caméra disposée, par l'intermédiaire d'une lumière de caméra (302), dans le cathéter, la caméra étant apte à fournir une ou plusieurs images de l'appareil de coupe rétractable.

8. Dispositif médical selon l'une quelconque des revendications 1 à 7, comprenant en outre un ou plusieurs fils électriques (134) configurés pour fournir un courant électrique à une ou plusieurs de l'au moins une surface de coupe du premier élément d'incision, le ou les fils électriques appliquant une force de sollicitation au premier élément d'incision,
facultativement le ou les fils électriques comprenant un ressort de torsion conçu pour appliquer la force de sollicitation au premier élément d'incision.

9. Dispositif médical selon l'une quelconque des revendications 1 à 8, comprenant en outre une tige d'actionnement (121) en communication avec l'extrémité distale de deuxième élément du deuxième élément d'incision, la tige d'actionnement étant configurée pour déplacer le premier élément d'incision et le deuxième élément d'incision.

10. Dispositif médical selon la revendication 9, comprenant en outre une chape (309) fixée à l'extrémité distale de deuxième élément du deuxième élément d'incision, la chape étant configurée pour recevoir la force provenant de la tige d'actionnement ou une force de sollicitation associée à la tige d'actionnement.

11. Dispositif médical selon l'une quelconque des revendications 1 à 10, comprenant en outre une gaine (130) configurée pour recouvrir de manière mobile l'ouverture d'incision.

12. Dispositif médical selon la revendication 11, dans lequel la gaine est configurée pour appliquer une force antagoniste à la force de sollicitation due au ou aux fils électriques dans le cas où la gaine recouvre au moins partiellement l'ouverture d'incision.

13. Dispositif médical selon l'une quelconque des revendications 1 à 12, dans lequel l'au moins une surface de coupe du premier élément d'incision comprend une première surface de coupe intérieure (205A) et une deuxième surface de coupe intérieure (205B), la première surface de coupe intérieure et la deuxième surface de coupe intérieure étant toutes deux des électrodes, et une impédance entre la première surface de coupe intérieure et la deuxième surface de coupe intérieure indiquant qu'un matériau est en cours de coupe par la première surface de coupe intérieure et la deuxième surface de coupe intérieure.

14. Dispositif médical selon l'une quelconque des revendications 1 à 13, comprenant en outre un contrôleur (1000) coopérant avec l'ensemble d'incision ou l'appareil de coupe rétractable.

15. Dispositif médical selon la revendication 14, dans lequel le contrôleur est configuré pour assurer au moins l'une des fonctions suivantes : fournir une aspiration à la ou aux lumières d'aspiration, déplacer le premier élément d'incision et le deuxième élément d'incision entre la position rétractée et la position déployée, déplacer l'ensemble d'incision, fournir de l'énergie à l'ensemble d'incision, ou déplacer un ou plusieurs éléments de stabilisation.
